(19) **Europäisches Patentamt / European Patent Office / Office européen des brevets**

(11) **EP 4 527 996 A1**

(12) **EUROPEAN PATENT APPLICATION**
published in accordance with Art. 153(4) EPC

(43) Date of publication:
**26.03.2025 Bulletin 2025/13**

(21) Application number: **23807549.3**

(22) Date of filing: **12.05.2023**

(51) International Patent Classification (IPC):
*D01F 4/00* (2006.01)    *A61L 27/22* (2006.01)
*A61L 27/60* (2006.01)    *D02G 3/04* (2006.01)
*D04B 1/14* (2006.01)

(52) Cooperative Patent Classification (CPC):
**A61L 27/22; A61L 27/60; D01F 4/00; D02G 3/04; D04B 1/14**

(86) International application number:
**PCT/JP2023/017834**

(87) International publication number:
**WO 2023/223946 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.05.2022   JP 2022080627**
**31.10.2022   JP 2022174225**

(71) Applicant: **Teijin Limited**
**Osaka 530-0005 (JP)**

(72) Inventors:
• **SAKURAI Hiroshi**
  **Osaka-shi Osaka 530-0005 (JP)**
• **HINO Yasushi**
  **Osaka-shi Osaka 530-0005 (JP)**
• **ZHANG Yaqi**
  **Osaka-shi Osaka 530-0005 (JP)**

(74) Representative: **Hoffmann Eitle**
**Patent- und Rechtsanwälte PartmbB**
**Arabellastraße 30**
**81925 München (DE)**

(54) **FIBER SHEET STRUCTURE INCLUDING MULTIPLE WOUND YARN BUNDLE FORMED OF GELATIN FIBER BUNDLE AND THERMOPLASTIC RESIN FIBER BUNDLE**

(57)    [Problem]

An object of the present invention is to provide a fiber sheet structure including doubled yarn bundles made of gelatin fiber bundles, having a good texture, high tensile strength, and large breaking elongation compared to conventional gelatin fibers, and thermoplastic resin fiber bundles.

Furthermore, an object of the present invention is to provide a fiber sheet structure including doubled yarn bundles made of gelatin fiber bundles, having excellent cell adhesiveness for cell colonization and a slipperiness unlikely to cause inflammation on rubbing against biological tissue, and thermoplastic resin fiber bundles.

[Solution]

The fiber sheet structure includes a knit weave and has a cell-mounting front surface and a back surface, the average pore size of the back surface is smaller than the average pore size of the cell-mounting surface, the knit weave includes doubled yarn bundles made of gelatin fiber bundle and thermoplastic resin fiber bundle, the thickness of the fiber sheet structure is 30 to 5000 μm, the number of gelatin fibers constituting the gelatin fiber bundle is 5 to 60, the fineness of the gelatin fiber is 0.5 to 10 dtex, the total number of gelatin fiber bundles and thermoplastic resin fiber bundles in the doubled yarn bundle is 2 to 20, and the ratio of the number of gelatin fiber bundles to thermoplastic resin fiber bundles in the doubled yarn bundle is 5:1 to 1:5.

**EP 4 527 996 A1**

**Description**

[Technical Field]

The present invention relates to a fiber sheet structure including doubled yarn bundles made of gelatin fiber bundles and thermoplastic resin fiber bundles.

[Background Art]

Gelatin is denatured collagen obtained by treating triple helix molecules of collagen obtained from cow bones, cowhide, pigskin, etc. with acid or alkali and then extracting with hot water. Gelatin has low antigenicity when placed in a living organism, and is decomposed by all kinds of enzymes, thus exhibits much quicker bioabsorbability than conventional bioabsorbable materials, and therefore suitably used as a bioabsorbable material. Conventionally, gelatin has been handled in the form of powder, sheets or sponges and has been used primarily in food ingredients, photographic emulsions, pharmaceutical capsules, etc. However, recently, gelatin has attracted attention for the excellent biocompatibility (low antigenicity and high bioabsorbability), and is expected to be used in medical materials such as wound/burn treatment materials and artificial skin matrix materials. In vivo, cells perform their functions through three-dimensional interactions. Furthermore, when considering a scaffold material for in vitro three-dimensional cell culture, three-dimensional cell culture is one means for imitating a living organism in vitro, and higher order structure is desirable. From the above viewpoints, for medical applications and scaffold materials for cell culture, higher order structures such as woven fabrics, knitted fabrics, and braided cords are desirably prepared using long fibers (filament yarns) having high strength and breaking elongation.

For example, PTL 1 proposes spinning by extruding into air an aqueous solution containing gelatin and a water-soluble linear polymer such as polyethylene glycol. PTL 2 proposes a method for producing gelatin fibers by discharging a gelatin solution into a coagulation bath to form gel-like fibers, and then taking out and stretching the fibers and removing the remaining solution. PTL 3 proposes heating an aqueous gelatin solution to solate and spinning it in air, and then crosslinking it by immersing in a crosslinking agent solution. PTL 4 proposes a method for producing gelatin fibers by wet spinning a gelatin solution containing an amide compound and a halogen salt of an alkali metal or an alkaline earth metal to be extruded into an alcohol solution to coagulate gelatin yarn, and washing away the added components such as a crosslinking agent.

When higher order structures such as woven fabrics, knitted fabrics, and braided cords having high strength and breaking elongation are used as medical materials, it is required to allow cells to colonize and grow on the higher order structures.

[Citation List]

[Patent Literature]

[PTL 1] JP-A-2012-167397
[PTL 2] JP-A-2005-120527
[PTL 3] JP-A-2005-163204
[PTL 4] JP-A-2001-89929

[Summary of Invention]

[Technical Problem]

However, because of the difficulty of unwinding the wound long fibers (filament yarns) made of gelatin fibers obtained by the above-mentioned conventional techniques, it has not been possible to obtain filament yarns. In addition, gelatin fibers produced through a washing process to reduce the toxicity of added components such as crosslinking agents had too low tensile strength and breaking elongation to obtain higher order structures such as knitted fabrics, woven fabrics, and braided cords.

In addition, gelatin generally has low cell adhesiveness, and thus has a problem of difficulty in colonizing and culturing of cells in a short period of time. In contrast, though having high cell adhesiveness, collagen has a problem that its decomposition can be achieved by only specific enzymes, which causes a long decomposition time in vivo. Furthermore, although use as a material for wound/burn treatment and as a matrix material for artificial skin requires slipperiness and moderate strength which are unlikely to cause inflammation even on rubbing against biological tissue, and on one hand, gelatin sponges and gelatin sheets have excellent slipperiness; on the other hand, they have a problem of low strength. Although higher order structures made of thermoplastic fibers have strength enough to withstand sliding, they have problems such as poor slipperiness causing inflammation on rubbing against biological tissue and inflammation-causing acids produced by the decomposition of thermoplastic fibers.

Therefore, an object of the present invention is to provide a fiber sheet structure including doubled yarn bundles made of gelatin fiber bundles, having a good texture, high tensile strength, and large breaking elongation compared to conventional gelatin fibers, and thermoplastic resin fiber bundles.

Furthermore, an object of the present invention is to provide a fiber sheet structure including doubled yarn bundles made of gelatin fiber bundles, having excellent cell adhesiveness for cell colonization and a slipperiness unlikely to cause inflammation on rubbing against biological tissue, and thermoplastic resin fiber bundles.

[Solution to Problem]

Specific means for achieving the above object include the following embodiments.

(First Aspect)

Embodiment 1

A higher order structure containing a gelatin fiber bundle.

Embodiment 2

The higher order structure according to embodiment 1, wherein the gelatin fiber bundle has a tensile strength of 0.5 to 3.0 cN/dtex and a breaking elongation of 30 to 300%.

Embodiment 3

The higher order structure according to embodiment 1 or 2, wherein the gelatin fiber bundle has a swelling ratio of 110 to 350% when immersed in phosphate buffered saline at 50°C for 4 hours.

Embodiment 4

The higher order structure according to any one of embodiments 1 to 3 further comprising a thermoplastic resin fiber bundle, wherein the gelatin fiber bundle and the thermoplastic resin fiber bundle form a doubled yarn bundle.

Embodiment 5

The higher order structure according to embodiment 4, wherein the tensile strength of the doubled yarn bundle is 1.0 to 10.0 cN/dtex and the breaking elongation is 20 to 250%.

Embodiment 6

The higher order structure according to embodiment 4 or 5, wherein the swelling ratio of the doubled yarn bundle is 110 to 350% when immersed in phosphate buffered saline at 50°C for 4 hours.

Embodiment 7

The higher order structure according to any one of embodiments 1 to 6, wherein the higher order structure has a weight loss rate of 3 to 30% when immersed in phosphate buffered saline at 50°C for 4 hours.

Embodiment 8

The higher order structure according to any one of embodiments 1 to 7, wherein the higher order structure has a thickness of 30 to 5000 $\mu$m.

Embodiment 9

The higher order structure according to any one of embodiments 1 to 8, wherein the higher order structure has a porosity of 60 to 97%.

Embodiment 10

The higher order structure according to embodiment 4 or 5, wherein the thermoplastic resin is coated with a biodegradable resin.

Embodiment 11

The higher order structure according to any one of embodiments 1 to 10, wherein the higher order structure is in the form of a knitted fabric.

Embodiment 12

A laminated higher order structure comprising the higher order structure according to any one of embodiments 1 to 11 and a structure made of a biodegradable resin.

Embodiment 13

The laminated higher order structure according to embodiment 12, wherein the structure made of a biodegradable resin is a nonwoven fabric.

Embodiment 14

The laminated higher order structure according to embodiment 12 or 13, wherein the laminated higher order structure has a weight loss rate of 3 to 30% when immersed in phosphate buffered saline at 50°C for 4 hours.

Embodiment 15

The laminated higher order structure according to any one of embodiments 12 to 14, wherein the laminated higher order structure has a thickness of 30 to 5000 $\mu$m.

Embodiment 16

The laminated higher order structure according to any one of embodiments 12 to 15, wherein the laminated higher order structure has a porosity of 10 to 90%.

Embodiment 17

The higher order structure according to any one of embodiments 1 to **11,** wherein the higher order structure has an average friction coefficient of 0.15 to 0.60.

Embodiment 18

The laminated higher order structure according to any one of embodiments 12 to 16, the laminated higher order structure has an average friction coefficient of 0.15 to 0.60.

Embodiment 19

The higher order structure according to any one of embodiments 1 to **11,** wherein the higher order structure has an average deviation of friction coefficient of 0.40 to 1.80.

Embodiment 20

The laminated higher order structure according to any one of embodiments 12 to 16, wherein the laminated higher order structure has an average deviation of friction coefficient of 0.40 to 1.80.

(Second Aspect)

Embodiment 21

A fiber sheet structure,

the fiber sheet structure includes a knit weave and has a cell-mounting front surface and a back surface,
the average pore size of the back surface is smaller than the average pore size of the cell-mounting front surface,
the knit weave includes a doubled yarn bundle made of a gelatin fiber bundle and a thermoplastic resin fiber bundle,
the thickness of the fiber sheet structure is 30 to 5000 $\mu$m,
the number of gelatin fibers constituting the gelatin fiber bundle is 5 to 60, and the fineness of the gelatin fiber is 0.5 to 10 dtex,
the doubled yarn bundle has the total number of gelatin fiber bundles and thermoplastic resin fiber bundles of 2 to 20 and the ratio of the number of gelatin fiber bundles to the number of thermoplastic resin fiber bundles of 5:1 to 1:5.

Embodiment 22

The fiber sheet structure according to embodiment 21, wherein the fiber sheet structure is any one of the following (1) to (4):

(1) The structure is two-layer laminated, the front surface side layer and the back surface side layer have a knit weave, and the knit weave is a jersey stitch or plain stitch,
(2) The structure is three-layer laminated, the front surface side layer has a knit weave, the back surface side layer is a nonwoven fabric, an intermediate layer between the front surface side layer and the back surface side layer has a knit weave, the knit weave is a jersey stitch or plain stitch, and the nonwoven fabric is a structure containing gelatin fibers,
(3) The structure is two-layer laminated, the front surface side layer has a knit weave, the back surface side layer has a nonwoven fabric, the knit weave is a rib stitch, and the nonwoven fabric is a structure containing gelatin fibers,
(4) A structure with a knit weave consisting of a rib stitch.

Embodiment 23

The fiber sheet structure according to embodiment 21 or 22, wherein the average pore size of the cell-mounting front surface is 5 to 123 $\mu$m, the number of pores with a size of 150 $\mu$m or more on the cell-mounting front surface is 35% or less, and the number of pores in the knit weave is 600 pores/mm$^3$ or more.

Embodiment 24

The fiber sheet structure according to any one of embodiments 21 to 23, wherein the swelling ratio of the doubled yarn bundle is 110 to 350% when immersed in phosphate buffered saline at 50°C for 4 hours.

Embodiment 25

A fiber sheet structure according to any one of embodiments 21 to 24, wherein the tensile strength of the doubled yarn bundle is 1.0 to 20.0 cN/dtex and the breaking elongation is 20 to 250%.

Embodiment 26

The fiber sheet structure according to any one of embodiments 21 to 25, wherein the weight loss rate of the fiber sheet structure is 3 to 30% when immersed in phosphate buffered saline at 50°C for 4 hours.

[Advantageous Effects of Invention]

The fiber sheet structure of the present invention contains gelatin fiber bundles having a good texture, high tensile strength, and large breaking elongation. For this reason, the gelatin fiber bundles can be used to form a fiber sheet structure, and the fiber sheet structure of the present invention contains such fiber bundle-like gelatin as a component to be a medical material with excellent mechanical strength and biocompatibility (low antigenicity and high bioabsorbability), and can be suitably used as wound/burn treatment materials and artificial skin matrix materials, etc.

The fiber sheet structure of the present invention has excellent cell adhesiveness for cell colonization and cell cultivatability. Gelatin is a biocompatible material (low antigenicity and high bioabsorbability), and therefore can be suitably used as a scaffold material for three-dimensional cell culture, which is excellent in promoting cell differentiation, enhancing cell function, and colonizing after transplantation. Furthermore, the fiber sheet structure of the present invention in a wet state has softness and slipperiness similar to those of living tissue. For this reason, it can be suitably used as a material for treating wound/burn, a matrix material for artificial skin, etc., and is unlikely to cause inflammation on rubbing against living tissue.

[Description of Embodiments]

Hereinafter, embodiments of the present invention will be described in detail. However, the present invention is not limited to the following embodiments. In the following embodiments, components (including component steps, etc.) are not essential unless otherwise specified. The same applies to numerical values and their ranges, which do not limit the present invention.

First Embodiment

<higher order structure>

The higher order structure of the first embodiment includes gelatin fiber bundles.

The higher order structure of the first embodiment may be any structure containing gelatin fiber bundles, and is preferably a knitted fabric, a woven fabric, or a braided cord, more preferably a knitted fabric from the viewpoints of shrinkability (handleability), dimensional stability, mechanical properties, and having an appropriate space for cell proliferation.

The knitted fabric may curl when trimmed and immersed in the culture solution. The curling may be prevented by producing the knitted fabric using rib stitch or garter stitch, or sewing together two knitted fabrics placed to curl in line symmetry to produce a double knit. Double knitting not only prevents curling, but also has the advantage of improving cell cultivatability when used as a scaffold material due to likeliness that the highly hydrophilic nature of gelatin allows cell culture medium to be retained in the mesh of knitted fabrics.

The weight loss rate of the higher order structure of the first embodiment when immersed in phosphate buffered saline at 50°C for 4 hours is preferably 3 to 30%, more preferably 5 to 28%, further preferably 7 to 26%, particularly preferably 7 to 24%, and most preferably 7 to 22%. The above weight loss rate was calculated using the following formula by measuring the weight as follows: measuring the weight of 25 cm$^2$ of higher order structure, wrapping it in a 10 $\mu$m membrane filter, immersing it in phosphate-buffered saline at 50°C for 4 hours, pulling up the membrane filter containing the higher order structure from the phosphate-buffered saline and immersing it in water at room temperature for 30 minutes, absorbing the water with a Kimtowel, drying the membrane filter containing the higher order structure at 80°C for 24 hours, taking the higher order structure from the membrane filter, and measuring the weight.

Weight loss rate [%] = 100 - [100 $\times$ (weight after immersion)] / (weight before immersion)

The weight loss rate within the above range for the higher order structure of the first embodiment when immersed in phosphate-buffered saline at 50°C for 4 hours allows the structure to exhibit excellent cell adhesiveness and to suppress the canceration of proliferated cells. When cells strongly adhere to the higher order structure, the cells tend to grow in a two-dimensional form to be preferably used mainly as an implantable medical material, and when cells weakly adhere to the higher order structure, the cells tend to grow in a three-dimensional form such as a spheroid to be preferably used as a scaffold material for in vitro three-dimensional cell culture. Furthermore, enabled are prevention of gelatin from easily dissolving in vivo and, in addition, exhibition of excellent handleability when used as a medical material or a scaffold material for three-dimensional cell culture.

The weight loss rate can be controlled by subjecting the gelatin fiber bundle precursor to any of the following treatments (water-resistant treatments): thermal crosslinking, chemical crosslinking using a crosslinking agent, and physical crosslinking using ultraviolet rays, radiation, or electron beams.

The thickness of the higher order structure of the first embodiment is preferably 30 to 5000 $\mu$m, more preferably 50 to 3000 $\mu$m, further preferably 100 to 2000 $\mu$m, and particularly preferably 150 to 1000 $\mu$m. The thickness of the higher order structure less than 30 $\mu$m will cause the higher order structure to reduce the strength and thus be easily broken, which is not preferable. The thickness exceeding 5000 $\mu$m, though allowing the higher order structure to have sufficient strength for handling, causes problems when used as a scaffold material for cell culture, such as cell death of anoxia, etc. near the center of the thickness of the higher order structure. The thickness can be measured by attaching adhesive tape to the ends of a higher order structure cut into a 3 cm square to fix the higher order structure, and using a contact thickness meter.

The porosity of the higher order structure of the first embodiment is preferably 60 to 97%, more preferably 65 to 95%, further preferably 70 to 93%, and particularly preferably 75 to 92%. The porosity less than 60% will cause the higher order structure to significantly reduce the stretchability and a difficulty in, for example, sticking the higher order structure along the affected area. In contrast, the porosity exceeding 97% will cause the pores to be too large for cells to colonize on the higher order structure. The porosity is calculated using the following formula by measuring the thickness and weight of the higher order structure cut into a 5 cm square.

$$\text{Porosity (\%)} = 100 \times [1 - a \div \{(b + c) \div (b \div d + c \div e)\} \div (f \times 25)]$$

a: Weight (g) of higher order structure cut into a 5 cm square
b: Gelatin fiber bundle fineness (dtex)
c: Thermoplastic fiber bundle fineness (dtex)
d: Gelatin fiber bundle density (g/cm$^3$)
e: Thermoplastic fiber bundle density (g/cm$^3$)
f: Thickness of higher order structure (cm)

Note that, the following values are used for each density: gelatin fiber bundle density of 1.27 g/cm$^3$, thermoplastic fiber bundle density including polyglycolic acid fiber bundle density of 1.53 g/cm$^3$ and polylactic acid fiber bundle density of 1.25 g/cm$^3$. In addition, when the higher order structure is produced using only gelatin fiber bundles without using thermoplastic fiber bundles, the above c and e are not included in the calculation.

The higher order structure of the first embodiment can be produced by a known method used for manufacturing knitted fabrics, woven fabrics, braided cords, and the like.

The higher order structure of the first embodiment comes to exhibit tactility in a wet state similar to that of biological tissue by containing gelatin fiber bundles, and thus is characterized by exhibiting more slipperiness compared with a higher order structure made only of thermoplastic resin fiber bundles. The tactility of the thermoplastic resin fiber bundle may be closer to that of living tissue by coating the surface of the thermoplastic resin fiber bundle with collagen, gelatin, or the like.

Generally, a higher order structure made of a thermoplastic resin fiber bundle has a problem of great difficulty due to the hydrophobicity in impregnating with a cell culture medium. However, the higher order structure of the first embodiment comes to be easily impregnated with cell culture medium by containing gelatin fiber bundles having excellent hydrophilicity, resulting in easy colonization and proliferation of cells inside the higher order structure.

The higher order structure of the first embodiment will be described in detail below.

(Gelatin fiber bundle)

The number of gelatin fibers constituting the gelatin fiber bundle of the first embodiment is preferably 2 to 90, more preferably 3 to 60, and further preferably 4 to 50.

The fineness of the gelatin fiber bundle is preferably 10 to 600 dtex, more preferably 30 to 500 dtex, and further preferably 50 to 400 dtex. The fineness of the gelatin fiber bundle is a value measured in grams per 10000 m.

The number of fibers constituting the gelatin fiber bundle and the fineness of the gelatin fiber bundle within the above ranges enables improvement of the qualities such as the texture, stretchability, and dimensional stability of the higher order structure.

The fineness of the gelatin fiber bundle can be controlled by the number of fibers constituting the gelatin fiber bundle and the fineness of the gelatin fiber described later.

The tensile strength of the gelatin fiber bundle of the first embodiment is preferably 0.5 to 3.0 cN/dtex, more preferably 0.7 to 2.8 cN/dtex, and further preferably 1.0 to 2.5 cN/dtex.

The tensile strength of the gelatin fiber bundle is measured, for example, by using a Tensilon (RTC-1210A manufactured by Orientec Co., Ltd.).

The breaking elongation of the gelatin fiber bundle is preferably 30 to 300%, more preferably 30 to 250%, further preferably 40 to 200%, and particularly preferably 50 to 150%. The breaking elongation of the gelatin fiber bundle is measured using a Tensilon (RTC-1210A manufactured by Orientec Co., Ltd.).

The tensile strength and breaking elongation of the gelatin fiber bundle within the above ranges will prevent breakage of the fiber bundle during winding, unwinding, and preparing a higher order structure, and thus facilitate easy preparation of the higher order structure.

The tensile strength and breaking elongation of the gelatin fiber bundle can be controlled by the type of gelatin and spinning conditions described below.

The weight loss rate of the gelatin fiber bundle of the first embodiment when immersed in phosphate buffered saline at 50°C for 4 hours is preferably 3 to 30%, more preferably 5 to 28%, further preferably 7 to 26%, particularly preferably 7 to 24%, and most preferably 7 to 22%. The above weight loss rate was calculated using the following formula by measuring the weight as follows: measuring the weight of a 100 mm gelatin fiber bundle, wrapping it in a 10 μm membrane filter, immersing it in phosphate-buffered saline at 50°C for 4 hours, pulling up the membrane filter containing the gelatin fiber bundle from the phosphate-buffered saline and immersing it in water at room temperature for 30 minutes, absorbing the water with a Kimtowel, drying the membrane filter containing the gelatin fiber bundle at 80°C for 24 hours, taking the fiber bundle from the membrane filter, and measuring the weight.

Weight loss rate [%] = 100 - [100 × (weight after immersion)] / (weight before immersion)

The weight loss rate within the above range for the gelatin fiber bundle of the first embodiment when immersed in phosphate-buffered saline at 50°C for 4 hours allows the bundle to exhibit excellent cell adhesiveness and to suppress the canceration of proliferated cells. When cells strongly adhere to the higher order structure, the cells tend to grow in a two-dimensional form to be preferably used mainly as an implantable medical material, and when cells weakly adhere to the higher order structure, the cells tend to grow in a three-dimensional form such as a spheroid to be preferably used as a scaffold material for in vitro three-dimensional cell culture. Furthermore, enabled are prevention of gelatin from easily dissolving in vivo and, in addition, exhibition of excellent handleability when used as a medical material or a scaffold material for three-dimensional cell culture.

The weight loss rate when a gelatin fiber bundle is immersed in phosphate buffered saline at 50°C for 4 hours can be controlled by the degree of insolubilization treatment described later.

The swelling ratio of the gelatin fiber bundle of the first embodiment when immersed in phosphate buffered saline at 50°C for 4 hours is preferably 110 to 350%, more preferably 120 to 300%, and further preferably 130 to 300%. The swelling ratio is a value calculated as follows: by measuring the fiber diameter of the gelatin fibers constituting the gelatin fiber bundle with an optical microscope RH2000 manufactured by HIROX, immersing the gelatin fiber bundle in phosphate buffered saline at 50°C for 4 hours, then pulling up the gelatin fiber bundle from the phosphate buffered saline, measuring the fiber diameter of the gelatin fibers in a wet state, and calculating the swelling ratio using the following formula.

Swelling ratio [%] = 100 × (fiber diameter after immersion) / (fiber diameter before immersion)

The swelling ratio within the above range for the gelatin fiber bundle of the first embodiment when immersed in phosphate-buffered saline at 50°C for 4 hours allows the bundle to exhibit excellent cell adhesiveness and to suppress the canceration of proliferated cells. When cells strongly adhere to the higher order structure, the cells tend to grow in a two-dimensional form to be preferably used mainly as an implantable medical material, and when cells weakly adhere to the higher order structure, the cells tend to grow in a three-dimensional form such as a spheroid to be preferably used as a scaffold material for in vitro three-dimensional cell culture. Furthermore, enabled are prevention of gelatin from easily dissolving in vivo and, in addition, exhibition of excellent handleability when used as a medical material or a scaffold material for three-dimensional cell culture.

Examples of the cross-sectional shape of the gelatin fibers constituting the gelatin fiber bundle include solid circle, hollow, flat, star, triangle, solid circle+fin, hollow+fin. As a scaffold material for three-dimensional cell culture, preferably used a modified cross-section yarn with a shape, such as flat, star, and triangle rather than solid circle.

When measuring the above-mentioned tensile strength, breaking elongation, weight loss rate, and swelling ratio of the gelatin fiber bundle from the higher order structure, a measurement sample was prepared by cutting the higher order structure with scissors, pinching the yarn end, and pulling out the yarn by 5 cm or more.

(Method for producing higher order structure)

A method for producing the higher order structure of the first embodiment will be described below though not particularly limited thereto.

The method for producing a higher order structure preferably includes the following steps 1 and 2, and step 3-1 or step 3-2.

(Step 1) A step of extruding a dope, made of a gelatin solution containing 30 to 70 wt% of a gelatin raw material, from a multi-hole spinneret into a fiber form, at a temperature 1 to 15°C higher than the sol-gel transition temperature of the gelatin dope;

(Step 2) A step of winding the extruded gelatin fiber around a bobbin to prepare a gelatin fiber bundle precursor;

(Step 3-1) A step of insolubilizing the gelatin fiber bundle precursor and then weaving it to prepare a higher order structure;

(Step 3-2) A step of preparing a higher order structure by weaving the gelatin fiber bundle precursor and then insolubilizing it.

The step 2 includes a conveying step of conveying the extruded gelatin fiber along the peripheral surface of a conveying roller before being wound around a bobbin, wherein the extruded gelatin fiber preferably has a gelatin fiber moisture content of 5 to 25 wt% on arriving at the first conveying roller at which the extruded gelatin fiber arrives first.

The moisture content of the gelatin fiber bundle precursor is preferably 5 to 20 wt%.

The step 2 includes a step of applying a mixed solution of at least one selected from ethylene glycol, polyethylene glycol, and glycerin and a lower alcohol to the extruded gelatin fibers before being wound around a bobbin.

In the step 1, preferably, the gelatin has a jelly strength of 100 to 350 g, and the gelatin dope has a sol-gel transition temperature of 15 to 60°C.

The higher order structure of the first embodiment can be produced by extruding fibrously a dope made of a gelatin solution from a multi-hole spinneret, winding the extruded gelatin fiber around a bobbin to prepare a gelatin fiber bundle precursor, insolubilizing the gelatin fiber bundle precursor to produce a gelatin fiber bundle, and then weaving the gelatin fiber bundle. Alternatively, as described above, higher order structure made of gelatin fiber bundles may be produced by preparing a fiber bundle precursor, weaving the fiber bundle precursor, and then insolubilizing it.

Note that, the term "weaving" refers to the production of a woven fabric, knitted fabric, or braided cord from fiber bundles or fiber bundle precursors.

The gelatin raw material constituting the gelatin solution is obtained as a single molecular chain by unraveling the triple helix of collagen derived from cow bones, cowhide, pigskin, birds, and fish. Methods for producing such a gelatin raw material include an acid treatment method and a lime treatment method, and the gelatin raw material used in the first embodiment may be a gelatin raw material produced by either method, or may be a commercially available gelatin raw material. In addition, commercially available gelatin raw materials undergo various purification steps before being extracted in the manufacturing process, thus contain few components other than proteins, and generally have a composition of 85% or more protein, 8 to 14% water, 2% or less ash, and 1% or less other components (lipids, polysaccharides, etc.), and such general gelatin raw materials can also be used in the first embodiment. Although gelatin has an amino acid composition almost the same as that of collagen due to a heat-denatured material of collagen, the composition is highly specific compared to other proteins: glycine accounts for about one-third of the total and is repeated as one in every three in amino acid sequence. Collagen and gelatin can be distinguished from each other, for example, by observing a signal at 31.5 degrees obtained by wide-angle X-ray measurement. This signal at 31.5 degrees is a signal derived from the triple helical structure of collagen, and when the triple helical structure characterizing collagen is unraveled to become gelatin, this signal disappears.

In addition, the gelatin raw material has a jelly strength, highly correlated with the molecular weight, which is not particularly limited, of preferably 100 to 350 g, more preferably 150 to 320 g, and further preferably 220 to 310 g, from the viewpoint of spinnability. The jelly strength is a load (g) required to press down 4 mm the surface of the gelatin solution prepared by cooling a 6.67% gelatin solution at 10°C for 17 hours, with a plunger having a diameter of 1/2 inch (12.7 mm), using a small tabletop tester EZ-SX manufactured by Shimadzu Corporation.

The jelly strength of the gelatin raw material within the above range can prevent a significant decrease in the spinnability of the gelatin fiber, and further suppressing the moisture retention rate in the fiber can prevent breakage of the fiber during winding.

The content of the gelatin raw material in the gelatin solution is preferably 30 to 70 wt%, and more preferably 40 to 60 wt%. The content of the gelatin raw material in the gelatin solution within the above range can prevent a significant decrease in the spinnability of the gelatin fiber, and further can facilitate degassing bubbles generated during the gelatin solution production process, thereby preventing yarn breakage during spinning.

The solvent for dissolving the gelatin raw material is water, and (polyhydric) alcohols, dimethyl sulfoxide, etc. may be added to adjust the viscosity, if necessary.

The sol-gel transition temperature of the gelatin solution is preferably 15 to 60°C, more preferably 20 to 60°C, further preferably 20 to 55°C, and particularly preferably 20 to 50°C. The sol-gel transition temperature is measured, for example, by using a rheometer ARES manufactured by TA Instruments.

The sol-gel transition temperature within the above range can prevent strengthening of the adhesiveness, thus improve the handleability during the production of the gelatin fiber, and further, prevent adhesion of the wound gelatin fiber. In addition, can be prevented the thermal decomposition of gelatin at the spinning temperature, and possible occurrence of yarn breakage.

The gelatin fiber bundle precursor is produced by extruding fibrously a dope, made of gelatin raw material and a solvent such as water, from a multi-hole spinneret into the air, and by moderately orienting the molecular chains of the triple helix molecules of collagen loosened by drawing just below the spinneret, while moderately controlling the moisture content in the fibers. Controlling the degree of orientation of the molecular chains of the loosened collagen triple helix molecules and the moisture content in the fibers allow the tensile strength and breaking elongation of the yarn to increase, and at the same time, enables suppression of adhesion between the fibers.

The spinning method of extruding fibrously the dope into the air from a multi-hole spinneret may be any of a dry method, a semi-dry method, and a wet method, among which the dry method is preferable for preventing the formation of voids on the surface and inside of the fiber during the process of drying and removing the solvent and thus suppressing reduction of the tensile strength and breaking elongation of the gelatin fiber. The dry spinning temperature is preferably 1 to 25°C higher than the sol-gel transition temperature of the dope, and more preferably 5 to 20°C higher. Within the above range, a significant decrease in spinnability can be prevented.

In the production of the gelatin fiber bundle precursor used in the first embodiment, the moisture content in the fibers is preferably reduced rapidly just after spinning to a range of 5 to 25 wt%. The moisture content in the fiber within the above range enables suppression of the stickiness, and thus prevention of adhesion among the gelatin fibers discharged from the multi-hole spinneret and sticking of the gelatin fibers to rollers during the producing step, and the gelatin fiber precursor wound around the bobbin to be easily unwound from the bobbin. Furthermore, enabled are suppression of significant decrease in the elongation of the yarn, and thus prevention of yarn breakage during the step of producing gelatin fiber bundles and during the process of producing a higher order structure while rewinding the bundle from the bobbin.

There may be included a conveying step of conveying the gelatin fibers extruded from the spinneret along the peripheral surface of a conveying roller before winding the gelatin fibers around a bobbin.

The moisture content of the gelatin fiber is preferably 5 to 25 wt%, and more preferably 5 to 20 wt% on arriving at the first conveying roller at which the extruded gelatin fiber arrives first. The above moisture content is obtained by winding a gelatin yarn around a first conveying roller and measuring 0.5 g of the wound fiber at 150°C in an N2 atmosphere using a Karl Fischer moisture content meter (EV-2010 manufactured by HIRANUMA Co., Ltd.).

The moisture content within the above range enables prevention of sticking of the gelatin fibers to the conveying rollers, and suppression of a significant decrease in the elongation of the yarn, and thus prevention of yarn breakage.

The moisture content can be controlled by drying with dry air or dry hot air.

In order to prevent adhesion among the fibers due to contact, grooved guide rollers are preferably used as the conveying rollers.

The moisture content of the gelatin fiber bundle precursor of gelatin fibers wound around a bobbin is preferably 5 to 20 wt%, and more preferably 5 to 15 wt%. The above moisture content is obtained by measuring 0.5 g of the fiber bundle precursor wound around a bobbin at 150°C in an N2 atmosphere using a Karl Fischer moisture content meter (EV-2010 manufactured by HIRANUMA Co., Ltd.).

The moisture content within the above range enables the gelatin fiber precursor to be easily unwound from the bobbin, and suppression of a significant decrease in the elongation of the yarn, and thus prevention of yarn breakage during the process of producing a higher order structure while rewinding the yarn from the bobbin.

The moisture content can be controlled by drying with dry hot air or the like. In addition, after passing through the first conveying roller, the fiber bundle can be dried timely by a winder.

The gelatin fiber bundle used in the first embodiment is preferably produced at a spinning draft (discharge linear speed/winding speed) in the range of 0.01 to 2, more preferably 0.05 to 1.5. The value within the above range enables the molecular chains of the loosened collagen triple helix molecules to have sufficient orientation to prevent a decrease in tensile strength, and the fiber diameter to be prevented from becoming too thick, and the moisture content of the gelatin fibers to be sufficiently reduced before arriving at the first conveying roller, and as a result, the gelatin fibers to be prevented from sticking to the roller. Furthermore, enabled are suppression of significant decrease in the breaking elongation, and thus prevention of yarn breakage during the step of producing gelatin fiber bundles and during the process of producing a higher order structure while rewinding the bundle from the bobbin.

There may be included a step of applying an oily agent to the gelatin fibers extruded from the spinneret before being wound around a bobbin. The surface of the fibers is applied with an oily agent preferably after the moisture content in the fibers reaches the range of 5 to 20 wt%. Applying an oily agent to the fiber surface enables multiple gelatin fibers to be bundled together and handled as a gelatin fiber bundle. The application of the oily agent significantly improves the handleability of the yarn during the step of producing gelatin fiber bundles and during the process of producing a higher order structure while rewinding the yarn from the bobbin. Since the higher order structure of the first embodiment is used as a medical material, a biocompatible oily agent is preferably used, such as ethylene glycol, polyethylene glycol, or glycerin. These compounds are preferably used by diluting with a lower alcohol such as ethanol, and applied as a mixed solution to the fiber surface, and then the lower alcohol is removed by drying. The oily agent may be removed by washing after the production of the higher order structure, or may be left as applied. The amount of oily agent applied has no restriction as long as allowing a plurality of gelatin fibers to be bundled into a fiber bundle, and is preferably, for example, 0.5 to 5 wt%.

The cross-sectional shape of the gelatin fibers may be any shape. Examples of fiber cross-sectional shapes include solid circle, hollow, flat, star, triangle, solid circle+fin, hollow+fin. When used as a three-dimensional cell culture scaffold material, irregular cross-section yarns having irregular cross-sections such as flat, star, or triangle may be preferable to solid circle.

The tensile strength of the gelatin fiber is preferably 0.5 to 3 cN/dtex, more preferably 0.7 to 2.8 cN/dtex, and further preferably 1.0 to 2.5 cN/dtex. The tensile strength of the gelatin fiber is measured by taking out a single yarn from the gelatin fiber bundle precursor and using, for example, Tensilon (RTC-1210A manufactured by Orientec Co., Ltd.).

The breaking elongation of the gelatin fiber is preferably 30 to 250%, more preferably 40 to 200%, and further preferably 50 to 150%. The breaking elongation of the gelatin fiber is measured by taking out a single yarn from a gelatin fiber bundle and using, for example, Tensilon (RTC-1210A manufactured by Orientec Co., Ltd.).

The tensile strength and breaking elongation of the gelatin fiber within the above ranges enable the gelatin fiber to withstand the production of gelatin fiber bundles and the production of higher order structure.

The step of insolubilizing the gelatin fiber bundle precursor may be either of insolubilizing the gelatin fiber bundle precursor to produce gelatin fiber bundles, or weaving the fiber bundle precursor and then insolubilizing to produce a higher order structure made of gelatin fiber bundles. The insolubilization (treatment to make the material insoluble in water) may be performed by any of the following methods: thermal crosslinking, chemical crosslinking using a crosslinking agent, and physical crosslinking using ultraviolet light, radiation, or electron beams, or a combination of these methods may be used as appropriate. When thermal crosslinking is performed, it may be performed in air or under vacuum. The temperature during thermal crosslinking is preferably in the range of 110 to 200°C, more preferably 120 to 190°C, and further preferably 130 to 180°C. If the temperature is lower than 100°C, crosslinking will be insufficient, and the gelatin will dissolve in water in a wet state, making it unusable as a scaffold material. If the temperature is higher than 200°C, the gelatin will thermally decompose, causing a significant decrease in fiber strength, which is not preferable. The degree of crosslinking can be changed timely by the combination of the heat treatment temperature and the heat treatment time, which is preferably 3 to 48 hours and from the viewpoint of productivity, is preferably 4 to 24 hours. Chemical crosslinking using a crosslinking agent may induce formation of pores in the fibers during the washing process to remove the crosslinking agent in the fibers, which may reduce the tensile strength and breaking elongation of the gelatin fibers. In this case, yarn breakage may occur during the process of preparing a higher order structure from the insolubilized gelatin fiber bundles. For this reason, the insolubilization treatment by chemical crosslinking is preferably performed after weaving the fiber bundle precursor. Examples of crosslinking agents used in chemical crosslinking include glutaraldehyde and hexamethylene diisocyanate.

The higher order structure produced in the first embodiment may be immersed in, for example, a 20 to 80% glycerin aqueous solution. Immersing in an aqueous glycerin solution allows the higher order structure to produce a medical material having better followability to the affected area and a moist texture.

The higher order structure of the first embodiment may contain an additive. Examples of the additive include polyacids such as (anhydrous) citric acid and (anhydrous) maleic acid, magnetic particles such as gamma-ferric oxide, inorganic substances such as hydroxyapatite, endogenous proteins that promote cell proliferation and differentiation, and collagen. These additives may be applied on the higher order structure, or may be applied on the gelatin fiber bundles, gelatin fiber bundle precursors, or gelatin fibers, or may be added to the gelatin raw material.

(doubled yarn bundle)

The higher order structure of the first embodiment may further include thermoplastic resin fiber bundles, and the thermoplastic resin fiber bundles may form doubled yarn bundles with gelatin fiber bundles.

The thermoplastic resin in the thermoplastic resin fiber bundle is not limited as long as being used as a medical material, and examples thereof include polyethylene terephthalate (PET), polylactic acid (PLLA, PLLDA), poly-$\varepsilon$-caprolactone (PCL), (LA-CL) copolymers thereof, polyglycolic acid (PGA), copolymers of glycolic acid and lactic acid (PLGA), and polyglutamic acid. Among these, polylactic acid (PLLA, PLLDA), copolymer of glycolic acid and lactic acid (PLGA), polyglycolic acid (PGA), or polyglutamic acid is preferred from the viewpoint of unlikeliness to cause inflammation due to relatively mild acid production by decomposition of the thermoplastic resin fiber bundle.

The number of thermoplastic resin fibers constituting the thermoplastic resin fiber bundle is preferably 5 to 70, more preferably 7 to 50, and further preferably 10 to 30.

The number within the above range enables the gelatin-containing higher order structure to significantly improve the strength, which is preferable.

The total number of gelatin fibers and thermoplastic resin fibers constituting the doubled yarn bundle is preferably 10 to 300, more preferably 20 to 280, and further preferably 25 to 250.

The number within the above range enables the gelatin-containing higher order structure to have not only high strength but also appropriate slipperiness under wet conditions, and thus to be embedded or wrapped around a sliding portion, for example, around cartilage.

The fineness of the thermoplastic resin fiber bundle is preferably 10 to 100 dtex, more preferably 15 to 60 dtex, and further preferably 20 to 50 dtex.

The fineness within the above range enables the higher order structure to improve the qualities, such as texture, stretchability, and dimensional stability, from which viewpoint it is preferable.

The fineness of the doubled yarn bundle is preferably 20 to 800 dtex, more preferably 40 to 700 dtex, and further preferably 50 to 600 dtex.

The fineness within the above range enables the gelatin-containing higher order structure to have high strength and improve qualities such as texture, stretchability, and dimensional stability, and in addition, to have appropriate slipperiness under wet conditions that allows application to sliding parts such as the areas around cartilage, which is preferable.

The tensile strength of the doubled yarn bundle is preferably 1.0 to 20.0 cN/dtex, more preferably 2.0 to 18.0 cN/dtex, further preferably 3.0 to 15.0 cN/dtex, and particularly preferably 4.0 to 12.0 cN/dtex. The tensile strength of the doubled yarn bundle is measured, for example, by using a Tensilon (RTC-1210A manufactured by Orientec Co., Ltd.).

The breaking elongation of the doubled yarn bundle is preferably 20 to 250%, more preferably 20 to 200%, further preferably 20 to 150%, and particularly preferably 25 to 150%. The breaking elongation of the doubled yarn bundle is measured using a Tensilon (RTC-1210A manufactured by Orientec Co., Ltd.).

The tensile strength and breaking elongation of the doubled yarn bundle within the above ranges enables prevention of yarn breakage during winding and unwinding of the doubled yarn bundle and production of a higher order structure, and thus the higher order structure to be easily produced.

The weight loss rate of the doubled yarn bundle of the first embodiment when immersed in phosphate buffered saline at 50°C for 4 hours is preferably 3 to 30%, more preferably 5 to 28%, further preferably 7 to 26%, particularly preferably 7 to 24%, and most preferably 7 to 22%. The above weight loss rate was calculated using the following formula by measuring the weight as follows: measuring the weight of a 100 mm doubled yarn bundle, wrapping it in a 10 $\mu$m membrane filter, immersing it in phosphate-buffered saline at 50°C for 4 hours, pulling up the membrane filter containing the doubled yarn bundle from the phosphate-buffered saline and immersing it in water at room temperature for 30 minutes, absorbing the water with a Kimtowel, drying the membrane filter containing the doubled yarn bundle at 80°C for 24 hours, taking the doubled yarn bundle from the membrane filter, and measuring the weight.

Weight loss rate [%] = 100 - [100 $\times$ (weight after immersion)] / (weight before immersion)

The weight loss rate within the above range for the doubled yarn bundle of the first embodiment when immersed in phosphate-buffered saline at 50°C for 4 hours allows the bundle to exhibit excellent cell adhesiveness and to suppress the canceration of proliferated cells. When cells strongly adhere to the higher order structure, the cells tend to grow in a two-dimensional form to be preferably used mainly as an implantable medical material, and when cells weakly adhere to the higher order structure, the cells tend to grow in a three-dimensional form such as a spheroid to be preferably used as a scaffold material for in vitro three-dimensional cell culture. Furthermore, enabled are prevention of gelatin from easily dissolving in vivo and, in addition, exhibition of excellent handleability when used as a medical material or a scaffold material for three-dimensional cell culture.

The weight loss rate when a doubled yarn bundle is immersed in phosphate buffered saline at 50°C for 4 hours can be controlled by the degree of insolubilization treatment described later.

The swelling ratio of the doubled yarn bundle of the first embodiment when immersed in phosphate buffered saline at 50°C for 4 hours is preferably 110 to 350%, more preferably 120 to 300%, and further preferably 130 to 300%. The swelling ratio is a value calculated as follows: by measuring the bundle diameter of the doubled yarn bundle with an optical microscope RH2000 manufactured by HIROX, immersing the doubled yarn bundle in phosphate buffered saline at 50°C for 4 hours, then pulling up the doubled yarn bundle from the phosphate buffered saline, measuring the bundle diameter of the doubled yarn bundle in a wet state, and calculating the swelling ratio using the following formula.

Swelling ratio [%] = 100 $\times$ (diameter of doubled yarn bundle after immersion) / (diameter of doubled yarn bundle before immersion)

The swelling ratio within the above range for the doubled yarn bundle of the first embodiment when immersed in phosphate-buffered saline at 50°C for 4 hours allows the bundle to suppress the canceration of proliferated cells and prevent gelatin from easily dissolving in vivo, and further to exhibit excellent handleability when used as a medical material or a scaffold material for three-dimensional cell culture.

When measuring the above-mentioned tensile strength, breaking elongation, weight loss rate, and swelling ratio of the doubled yarn bundle from the higher order structure, a measurement sample was prepared by cutting the higher order structure with scissors, pinching the yarn end, and pulling out the yarn by 5 cm or more.

The cross-sectional shape of the thermoplastic resin fibers constituting the thermoplastic resin fiber bundle may be any shape, and examples thereof include solid circle, hollow, flat, star, triangle, solid circle+fin, hollow+fin. As a scaffold material for three-dimensional cell culture, a modified cross-section may be preferable to a solid circle cross-section.

The thermoplastic resin fibers used in the higher order structure of the first embodiment may contain an additive. Examples of the additive include polyacids such as (anhydrous) citric acid and (anhydrous) maleic acid, magnetic particles such as gamma-ferric oxide, inorganic substances such as hydroxyapatite, endogenous proteins that promote cell proliferation and differentiation, collagen, and pharmaceutical agents such as anticancer drugs. These additives may be applied on the thermoplastic resin fibers.

An example of a method for coating a thermoplastic resin fiber bundle with a biodegradable resin such as collagen is as follows: removing the oily agent from the thermoplastic fiber bundle in a cleaning bath while rewinding, and drying, then immersing the thermoplastic fiber bundle in a collagen solution to coat the thermoplastic fiber bundle with collagen, drying, and winding up. Collagen is easily heat-denatured, and thus is preferably applied at a temperature of 40°C or lower, preferably 30°C or lower, and more preferably 25°C or lower and dried. The coating treatment and drying treatment are not well performed at too low a temperature, resulting in coating impossible due to freeze of the collagen solution or insufficient drying, and thus are preferably performed at 0°C or higher, preferably 5°C or higher, and more preferably 10°C or higher. The collagen solution is preferably an acid solution with concentration of 0.005 to 3 wt%. The concentration of the collagen solution less than 0.005 wt% makes the collagen unable to bind the thermoplastic resin fiber bundles, and thus handling of the fiber bundles difficult. **In** contrast, the concentration exceeding 3 wt% makes the thermoplastic resin fiber bundle hard and the handleability significantly poor. The collagen solution concentration is more preferably 0.01 to 2 wt%. The collagen-coated thermoplastic fiber bundle may be heat-treated at 100 to 150°C or chemically treated to make the coating collagen insoluble in water.

Due to the property of having highly excellent cell adhesiveness, collagen has an excellent characteristic, taking an example of a higher order structure made of collagen-coated thermoplastic resin fiber bundles and gelatin fiber bundles, of fibrously colonizing and culturing cells without dropping cells from the gaps between the fibers. In addition, since the collagen coating allows the texture to be similar to that of living tissue, there is another characteristic of excellent slipperiness under wet conditions.

In addition to collagen, other biodegradable resins include bioderived resins such as gelatin, and synthetic polymers such as polyglycolic acid (PGA), polylactic acid (PLA), lactic acid-glycolic acid copolymer (PLGA), and polyglutamic acid.

The ratio of the gelatin fiber bundles to the thermoplastic resin fiber bundles in the doubled yarn bundle (fineness of the gelatin fiber bundles : fineness of the thermoplastic resin fiber bundles) is 1:5 to 5:1.

(Production method of doubled yarn bundle)

The thermoplastic resin fiber of the first embodiment can be produced by melt spinning, and a commercially available thermoplastic resin fiber bundle can also be purchased for use.

The higher order structure including the doubled yarn bundle of the first embodiment is produced using a starting material of a doubled yarn bundle precursor made of a gelatin fiber bundle precursor and a thermoplastic resin fiber bundle. A doubled yarn bundle precursor made of a gelatin fiber bundle precursor and a thermoplastic resin fiber bundle can be produced from a plurality of fiber bundle precursors using a doubling machine.

Twisting may be performed by spirally winding the thermoplastic resin fiber bundle around the gelatin fiber bundle precursor, or by spirally winding the gelatin fiber bundle precursor around the thermoplastic resin fiber bundle.

Doubling may be performed by twisting the gelatin fiber bundle precursor and the thermoplastic resin fiber bundle both with oily agent as applied thereto, or by twisting the fiber bundles with each other by interlacing etc.

The doubled yarn bundle precursor can be processed to the doubled yarn bundle in a manner similar to the step of insolubilizing the gelatin fiber bundle precursor described above, and then the bundle is woven to produce a higher order structure. Alternatively, the doubled yarn bundle precursor may be woven and then insolubilized to prepare a higher order structure made of the doubled yarn bundle.

The higher order structure including the doubled yarn bundle of the first embodiment may be laminated with a sheet such as nonwoven fabric, knitted fabric, woven fabric. For example, in the case of laminating with a gelatin or collagen nonwoven fabric, the following method can be exemplified: a doubled yarn bundle precursor is woven, on which a nonwoven fabric is laminated by electrospinning or the like. The laminated gelatin or collagen nonwoven fabric can be water-insolubilized by a crosslinking treatment.

(Laminated higher order structure)

The higher order structure of the first embodiment may be a laminated higher order structure including a structure made of a biodegradable resin. Examples of biodegradable resins include bioderived resins such as gelatin and collagen, and synthetic polymers such as polyglycolic acid (PGA), polylactic acid (PLA), lactic acid-glycolic acid copolymer (PLGA), and polyglutamic acid. The structure to be laminated may be in the form of a nonwoven fabric, knitted fabric, woven fabric, etc., and a nonwoven fabric is preferred. The gaps in the higher order structure (the mesh of the knitted fabric) are much larger than the size of the cells. For this reason, when the higher order structure is used as scaffold materials for cell culture, the cells fall off through the gaps in the higher order structure and are unable to colonize, which requires to spend time for cell culture. **In** contrast, when a laminated higher order structure including a higher order structure and a structure made of a biodegradable resin is used as a scaffold material for cell culture, the structure made of the biodegradable resin captures the cells and colonize them in the laminated higher order structure to shorten the cell culture time. Examples of methods for producing a laminated higher order structure containing a higher order structure and a structure made of biodegradable resin are as follows: a method of spraying a nonwoven fabric onto the higher order structure by electrospinning, melt-blowing, or the like while rewinding a roll of the higher order structure and conveying the higher order structure, and then heat-pressed with a calendar roll or the like to wind up, and a method of laminating a structure made of biodegradable resin on a cut-out higher order structure and then heat-pressing in a batch. The temperature of the heat press is preferably 40 to 90°C, and the pressure is preferably 5 kPa to 3 MPa. The heat pressing temperature less than 40°C is likely to cause interlayer delamination between the higher order structures and the structure made of biodegradable resin constituting the laminated higher order structure, and the temperature higher than 90°C causes welding of the gelatin fibers constituting the higher order structure, impairing the stretchability that is a characteristic of the higher order structure of the first embodiment, which is not preferable. The heat press temperature is preferably 40 to 80°C. The heat press pressure less than 5 kPa is likely to cause interlayer delamination between the high-order structure and the structure made of biodegradable resin constituting the laminated high-order structure, and the pressure exceeding 3 MPa causes impairment of the stretchability, a characteristic of the higher order structure of the first embodiment, which is not preferable. The heat press pressure is more preferably 400 kPa to 2 MPa.

The weight loss rate of the laminated higher order structure of the first embodiment when immersed in phosphate buffered saline at 50°C for 4 hours is preferably 3 to 30%, more preferably 5 to 28%, and further preferably 7 to 26%.

The rate within the above range enables achievement of excellent cell adhesiveness, and suppression of canceration of proliferated cells. When cells strongly adhere to the higher order structure, the cells tend to grow in a two-dimensional form to be preferably used mainly as an implantable medical material, and when cells weakly adhere to the higher order structure, the cells tend to grow in a three-dimensional form such as a spheroid to be preferably used as a scaffold material for in vitro three-dimensional cell culture. Furthermore, enabled are prevention of gelatin from easily dissolving in vivo and, in addition, exhibition of excellent handleability when used as a medical material or a scaffold material for three-dimensional cell culture.

The thickness of the laminated higher order structure of the first embodiment is preferably 30 to 5000 $\mu$m, more preferably 50 to 3000 $\mu$m, further preferably 100 to 2000 $\mu$m, and particularly preferably 150 to 1000 $\mu$m. The thickness of the laminated higher order structure less than 30 $\mu$m will cause the laminated higher order structure to reduce the strength and thus be easily broken, which is not preferable. The thickness exceeding 5000 $\mu$m, though allowing the laminated higher order structure to have sufficient strength for handling, causes problems when used as a scaffold material for cell culture, such as cell death of anoxia, etc. near the center of the thickness of the laminated higher order structure. The thickness can be measured by attaching adhesive tape to the ends of a laminated higher order structure cut into a 3 cm square to fix the laminated higher order structure, and using a contact thickness meter.

The porosity of the laminated higher order structure of the first embodiment is preferably 60 to 97%, more preferably 65 to 95%, further preferably 70 to 93%, and particularly preferably 75 to 92%. The porosity less than 60% will cause the laminated higher order structure to significantly reduce the stretchability and difficulty, for example, for the laminated higher order structure to be stuck along the affected area. In contrast, the porosity exceeding 97% will cause the pores to be too large for cells to colonize on the higher order structure. The porosity is calculated using the following formula by measuring the thickness and weight of the laminated higher order structure cut into a 5 cm square.

$$\text{Porosity (\%)} = 100 \times [1 - a \div \{(b + c) \div (b \div d + c \div e)\} \div (f \times 25)]$$

 a: Weight (g) of the laminated higher order structure cut into a 5 cm square
 b: Gelatin fiber bundle fineness (dtex)
 c: Thermoplastic fiber bundle fineness (dtex)
 d: Gelatin fiber bundle density (g/cm$^3$)
 e: Thermoplastic fiber bundle density (g/cm$^3$)
 f: Thickness of laminated higher order structure (cm)

Note that, the following values are used for each density: gelatin fiber bundle density of 1.27 g/cm$^3$, thermoplastic fiber bundle density including polyglycolic acid fiber bundle density of 1.53 g/cm$^3$ and polylactic acid fiber bundle density of 1.25 g/cm$^3$. In addition, when the higher order structure is produced using only gelatin fiber bundles without using thermoplastic fiber bundles, the above c and e are not included in the calculation.

<Characteristics of higher order structure and laminated higher order structure>

The higher order structure and the laminated higher order structure of the first embodiment are excellent in texture, slipperiness and cell adhesiveness.

The texture can be evaluated based on the touch feeling when the higher order structure and the laminated higher order structure are touched with dry hands. The texture was evaluated by three-time repetition of pinching the higher order structure cut into a 5 cm square at both ends with both hands and pulling them, and pulling one pinched end upwards and the other end downwards. No stretchability is likely to cause a stiff texture, which makes it difficult to follow biological tissue, and thus a moderate stretchability is required.

The slipperiness can be evaluated using a friction tester (model number: KES-SE) manufactured by Kato Tech Co., Ltd. The average friction coefficient and the average deviation of the friction coefficient were measured by immersing in distilled water for 1 hour a sample of the higher order structure and the laminated higher order structure cut into 5 cm square, then fixing the sample on a measuring table with a metal frame, and running a 10 mm square silicon sensor over the sample at 1.0 mm/sec with a load of 50 g.

The average friction coefficient of the higher order structure and the laminated higher order structure of the first embodiment is preferably 0.05 to 0.60, more preferably 0.10 to 0.50, further preferably 0.15 to 0.45, and most preferably 0.20 to 0.40. Within the above range, the slipperiness is similar to that of living tissue, and inflammation is unlikely to occur on rubbing against living tissue.

The average deviation of the friction coefficient of the higher order structure and the laminated higher order structure of the first embodiment is preferably 0.40 to 1.60, more preferably 0.45 to 1.45, further preferably 0.50 to 1.40, and most preferably 0.60 to 1.30. Within the above range, the higher order structure of the first embodiment has softness and slipperiness similar to those of living tissue under wet conditions. For this reason, these can be suitably used as a material for treating wound/burn, a matrix material for artificial skin, etc., and are unlikely to cause inflammation on rubbing against living tissue, which is preferable.

The higher order structure and the laminated higher order structure containing the gelatin fiber bundle of the first embodiment have excellent cell adhesiveness for colonizing cells. Cell adhesiveness strongly correlates with cell cultivatability: strong cell adhesiveness allows cells to efficiently colonize in the higher order structure to exhibit excellent cell cultivatability. In contrast, low cell adhesiveness cannot allow cells to colonize well in the higher order structure, which tends to exhibit poor cell cultivatability.

Gelatin is a biocompatible material (low antigenicity and high bioabsorbability), and therefore can be suitably used as a scaffold material for three-dimensional cell culture, which is excellent in promoting cell differentiation, enhancing cell function, and colonizing after transplantation. Furthermore, the higher order structure and laminated higher order structure of the first embodiment has softness and slipperiness similar to those of living tissue under wet conditions. For this reason, these can be suitably used as a material for treating wound/burn, a matrix material for artificial skin, etc., and are unlikely to cause inflammation on rubbing against living tissue, which is preferable.

The cell adhesiveness and cell cultivatability of the higher order structure and laminated structure can be evaluated by statically culturing HeLa cells on the structure for three days and observing the fluorescently labeled cells under a fluorescence microscope.

Second Embodiment

<Configuration of fiber sheet structure>

The fiber sheet structure of the present invention includes a knit weave and has a cell-mounting front surface and a back surface, the average pore size of the back surface is smaller than the average pore size of the cell-mounting front surface, the knit weave includes a doubled yarn bundle made of gelatin fiber bundle and thermoplastic resin fiber bundle, the thickness of the fiber sheet structure is 30 to 5000 µm, the number of gelatin fibers constituting the gelatin fiber bundle is 5 to 30, the fineness of the gelatin fiber is 0.5 to 10 dtex, the total number of gelatin fiber bundles and thermoplastic resin fiber bundles in the doubled yarn bundle is 2 to 20, and the ratio of the number of gelatin fiber bundles to thermoplastic resin fiber bundles in the doubled yarn bundle is 5:1 to 1:5.

The fiber sheet structure of the present invention includes a knit weave. A knit weave is a fabric weave in which a loop is made, and the next loop is hooked onto the previous one, and this step is repeated to form a surface. The knit weave has weft knit and warp knit, and weft knit is a knit weave in which the knitted loop is arranged continuously in the weft direction, while warp knit is a knit weave in which the knitted loop is arranged continuously in the warp direction. The weft knit includes plain stitch (jersey stitch), rib stitch, and purl stitch, while the warp knit includes tricot/raschel.

The knit weave in the fiber sheet structure of the present invention includes a doubled yarn bundle made of gelatin fiber bundles and thermoplastic resin fiber bundles.

The number of gelatin fibers constituting the gelatin fiber bundle in the fiber sheet structure of the present invention is preferably 5 to 60, and more preferably 10 to 40.

The fineness of the gelatin fibers constituting the gelatin fiber bundles in the fiber sheet structure of the present invention is preferably 0.5 to 10 dtex, more preferably 1 to 8 dtex, and further preferably 2 to 5 dtex. The fineness of gelatin fiber is measured in grams per 10000 meters. The fineness of the gelatin fiber within the above range enables the gelatin fiber itself to be a thin and flexible fiber, and thus enables not only suppression of generation of fluff in the gelatin fiber bundle due to yarn breakage but also prevention of hardening of the gelatin fiber bundle.

The number of gelatin fibers constituting the gelatin fiber bundles in the fiber sheet structure of the present invention and the fineness of the gelatin fibers within the above ranges enables the fiber sheet structure to improve the qualities such as texture, stretchability, and dimensional stability.

The preferred ranges and measurement methods for the tensile strength and breaking elongation of the gelatin fiber bundles in the fiber sheet structure of the present invention are the same as the preferred ranges and measurement methods for the tensile strength and breaking elongation of the gelatin fiber bundles in the higher order structure of the first embodiment described above.

Examples of the cross-sectional shape of the gelatin fibers constituting the gelatin fiber bundle in the fiber sheet structure of the present invention include solid circle, hollow, flat, star, triangle, solid circle+fin, hollow+fin. As a scaffold material for three-dimensional cell culture, preferably used is a modified cross-section yarn with a shape, such as flat, star, and triangle rather than solid circle.

When measuring the tensile strength and breaking elongation of a gelatin fiber bundle in a fiber sheet structure, a measurement sample can be prepared by treating a gelatin fiber bundle under the same conditions as those for insolubilization in the fiber sheet structure production process, or by cutting the bundle with scissors, pinching the end of the yarn and pulling out the yarn by 5 cm or more.

The thermoplastic resin in the thermoplastic resin fiber bundle in the fiber sheet structure of the present invention is not limited as long as being used as a medical material, and examples thereof include polyethylene terephthalate (PET), polylactic acid (PLLA, PLLDA), poly-$\varepsilon$-caprolactone (PCL), (LA-CL) copolymers thereof, polyglycolic acid (PGA), copolymers of glycolic acid and lactic acid (PLGA), polyglutamic acid. Among these, polylactic acid (PLLA, PLLDA), copolymer of glycolic acid and lactic acid (PLGA), polyglycolic acid (PGA), or polyglutamic acid is preferred from the viewpoint of unlikeliness to cause inflammation due to relatively mild acid production by decomposition of the thermoplastic resin fiber bundle.

The number of thermoplastic resin fibers constituting the thermoplastic resin fiber bundle in the fiber sheet structure of the present invention is preferably 5 to 70, more preferably 7 to 50, and further preferably 10 to 30. The number within the above range enables the fiber sheet structure to significantly improve the strength, which is preferable.

The total number of gelatin fiber bundles and thermoplastic resin fiber bundles constituting the doubled yarn bundle in the fiber sheet structure of the present invention is preferably 2 to 20, and more preferably 2 to 10. The total number within the above range enables the number of times for doubling the gelatin fiber bundles and the thermoplastic resin fiber bundles to be reduced, and production efficiency to be improved.

In the fiber sheet structure of the present invention, the ratio of the number of gelatin fiber bundles to the number of thermoplastic resin fiber bundles in the doubled yarn bundle is preferably 5:1 to 1:5. The ratio within the above range enables the doubled yarn bundle to have not only sufficient strength to withstand the production of a fiber sheet structure but also slipperiness similar to that of living tissue, and thus inflammation on rubbing against living tissue to be less likely to occur. The number of gelatin fiber bundles and the number of thermoplastic resin fiber bundles in the doubled yarn bundle are more preferably 3:1 to 1:3.

In the fiber sheet structure of the present invention, the preferred ranges, measurement methods, and control methods for the tensile strength, breaking elongation, weight loss rate when immersed in phosphate buffered saline at 50°C for 4 hours, and the swelling ratio when immersed in phosphate buffered saline at 50°C for 4 hours of the doubled yarn bundle are the same as the preferred ranges, measurement methods, and control methods for the tensile strength, breaking elongation, weight loss rate when immersed in phosphate buffered saline at 50°C for 4 hours, and swelling ratio when immersed in phosphate buffered saline at 50°C for 4 hours of the doubled yarn bundle in the higher order structure of the first embodiment described above.

When measuring the above-mentioned tensile strength, breaking elongation, weight loss rate when immersed in phosphate buffered saline at 50°C for 4 hours, and swelling ratio when immersed in phosphate buffered saline at 50°C for 4 hours, of the doubled yarn bundle in the fiber sheet structure, a measurement sample was prepared by cutting the fiber sheet structure with scissors, pinching the yarn end, and pulling out the doubled yarn bundle by 5 cm or more.

The cross-sectional shape of the thermoplastic resin fibers constituting the thermoplastic resin fiber bundle in the fiber sheet structure of the present invention may be any shape, and examples thereof include solid circle, hollow, flat, star, triangle, solid circle+fin, hollow+fin. As a scaffold material for three-dimensional cell culture, a modified cross-section may be preferable to a solid circle cross-section.

The thermoplastic resin fibers used in the fiber sheet structure of the present invention may contain an additive, which is the same as the additive that may be contained in the thermoplastic resin fibers used in the higher order structure of the first embodiment described above.

The fiber sheet structure of the present invention has a cell-mounting front surface and a back surface, and the cell-mounting front surface is the surface on which cells are placed for cell culture and the cells penetrate therethrough, and refers to the upper surface when placed in a cell culture vessel.

In the fiber sheet structure of the present invention, the average pore size of the cell-mounting back surface is smaller than the average pore size of the cell-mounting front surface. This case has the following effect: the cell-mounting front surface, which does not hinder cell penetration, allows cells to penetrate and colonize inside the fiber sheet structure, and the mesh on the cell-mounting back surface, which is smaller than the average pore size of the cell-mounting front surface, prevents cells from slipping off.

In the fiber sheet structure of the present invention, the average pore size of the cell-mounting front surface is preferably 5 to 123 $\mu$m, and more preferably 10 to 115 $\mu$m. The average pore size of the cell-mounting front surface within the above range enables cells to easily penetrate from the front surface of the fiber sheet structure into inside of the structure, and the cells can be prevented from slipping off through the mesh (pores), allowing the cells to be colonized on the fiber sheet structure. The average pore size on the cell-mounting front surface of the fiber sheet structure of the present invention can be determined as follows: obtaining a 35× image (pixel count: 1920 × 1200) of a fiber sheet structure cut into a 5 cm square using a digital microscope (RH-2000, manufactured by Hirox Co., Ltd.), then binarizing the obtained image into pores and non-pores using the 2D measurement software "HRS-2D" attached to the microscope, using the same software to create a histogram of pore sizes at 10 $\mu$m intervals from 60 to 300 $\mu$m, and calculating the average pore size in accordance with the following formula using the class value and frequency obtained from this histogram.

$$\text{Average pore size (μm)} = \{\Sigma\ (\text{class value} \times \text{frequency})\} \div (\Sigma\ \text{frequency})$$

In the fiber sheet structure of the present invention, the average pore size of the cell mounting back surface is preferably 5 to 115 $\mu$m, and more preferably 7 to 110 $\mu$m.

In the fiber sheet structure of the present invention, the proportion of number of pores having a size of 150 $\mu$m or more on the cell-mounting front surface is preferably 35% or less, more preferably 25% or less, further preferably 20% or less, and particularly preferably 15% or less. The above case allows the mesh (pores) to prevent cells from slipping off and the cells to colonize on the fiber sheet structure. The proportion of number of pores with a size of 150 $\mu$m or more on the cell-mounting front surface of the fiber sheet structure of the present invention can be calculated using the histogram used to calculate the average pore size described above, by dividing the total frequency of pores with a size of 150 $\mu$m or more by the total frequency of pores with sizes of 60 to 300 $\mu$m.

The porosity of the fiber sheet structure of the present invention is preferably 60 to 97%, more preferably 65 to 95%, further preferably 70 to 93%, and particularly preferably 75 to 92%. The porosity less than 60% will cause the fiber sheet structure to exhibit significantly reduced stretchability, resulting in, for example, difficulty in adhering the fiber sheet structure to follow the affected area. In contrast, the porosity exceeding 97% will cause the pores to be too large for cells to colonize on the fiber sheet structure. The method for measuring the porosity of the fiber sheet structure of the present invention is the same as the method for measuring the porosity of the higher order structure of the first embodiment described above.

In the fiber sheet structure of the present invention, the number of pores in the knit weave is preferably 600 pores/mm$^3$ or more, preferably 1.5 × 10$^3$ pores/mm$^3$ or more, preferably 1.8 × 10$^3$ pores/mm$^3$ or more, and preferably 2.1 × 10$^3$ pores/mm$^3$ or more. The above case allows a large number of cells to be transported into the fiber sheet structure, and the cells to efficiently colonize in the fiber sheet structure. The number of pores in the knit weave of the fiber sheet structure of the present invention was calculated by the following formula using the average pore size and porosity obtained above.

$$\text{Number of holes/mm}^3 = a \times 0.01 \div (4\,/\,3 \times 3.14 \times (0.001 \times b\,/\,2)^3)$$

a: Porosity (%)
b: Pore size ($\mu$m)

The fiber sheet structure of the present invention is a structure of two-layer laminated, the cell-mounting front surface side layer and the back surface side layer have a knit weave, and the knit weave is preferably a jersey stitch or plain stitch. The jersey stitch or plain stitch has characteristics of highly flat and smooth surface due to the minimal unevenness of the fabric surface. In addition, a single layer jersey stitch or plain stitch usually curls to form a cylindrical sheet structure. Such a case will cause the fiber sheet structure to have not only difficulty in colonizing the cells uniformly but also poor handleability. However, the curling can be suppressed by laminating two sheet layers, upper one curling convexly upwards and the lower one curling convexly downwards, to make the stress acting on both layers about the same. Suppressing curling will allow the fiber sheet structure not only to colonize cells uniformly but also to improve the handleability. Furthermore, laminating two layers will allow the area for colonizing cells to increase and the frequency of cell slipping-off through the mesh to be significantly reduced, as a result, a fiber sheet structure to exhibit excellent cell cultivatability.

The fiber sheet structure of the present invention preferably has a three-layer laminated structure, the cell-mounting front surface side layer has a knit weave, the back surface side layer is a nonwoven fabric, an intermediate layer between the front surface side layer and the back surface side layer has a knit weave, the knit weave is a jersey stitch or plain stitch, and the nonwoven fabric contains gelatin fibers. Since a nonwoven fabric has no residual stress and does not curl, a knit weave in the intermediate layer between the front surface side layer and the back surface side layer makes the stresses acting on each layer about the same, resulting in suppression of curling. Suppressing curling will allow the fiber sheet structure not only to colonize cells uniformly but also to improve the handleability. In addition, the average pore size of a nonwoven fabric overwhelmingly smaller than that of a knitted structure enables cells that have penetrated from the cell-mounting front surface side to be retained within the fiber sheet structure, resulting in a fiber sheet structure to exhibit excellent cell cultivatability.

The fiber sheet structure of the present invention preferably has a two-layer laminated structure, the cell-mounting front surface side layer has a knit weave, the back surface side layer has a nonwoven fabric, the knit weave is a rib stitch, and the nonwoven fabric contains gelatin fibers. Rib stitch does not curl due to the same weave on the front and back surface. Since the nonwoven fabric does not curl, the structure in which the nonwoven fabric is laminated does not curl. This not only allows cells to colonize uniformly in the fiber sheet structure, but also improves handleability. Since the average pore size of a nonwoven fabric is overwhelmingly smaller than that of a knitted structure, cells that penetrate from the cell-mounting front surface side can be retained within the fiber sheet structure, resulting in a fiber sheet structure with excellent cell cultivatability. In addition, compared to a fiber sheet structure of two-layer laminated knit weave, there is a characteristic of thinner thickness due to a single layer of knit weave.

The fiber sheet structure of the present invention is preferably a knit weave of rib stitch. In the above case, the none-curling rib stitch not only enables cells to colonize uniformly in the fiber sheet structure, but also improves handleability. In addition, compared to a fiber sheet structure of two-layer laminated knit weave, there is a characteristic of thinner thickness due to a single layer of knit weave.

The thickness of the fiber sheet structure of the present invention is preferably 30 to 5000 $\mu$m, more preferably 50 to 3000 $\mu$m, further preferably 100 to 2000 $\mu$m, and particularly preferably 150 to 1000 $\mu$m. The fiber sheet structure in the above range allows the fiber sheet structure to exhibit high strength and to be less likely to break, and to prevent causing problems when used as a scaffold material for cell culture, such as cell death of anoxia, etc. near the center of the thickness of the fiber sheet structure. The thickness of the fiber sheet structure of the present invention can be measured by attaching adhesive tape to the ends of a fiber sheet structure cut into a 3 cm square to fix the fiber sheet structure, and using a contact thickness meter.

The preferred range, measurement method, and control method for the weight loss rate when the fiber sheet structure of the present invention is immersed in phosphate buffered saline at 50°C for 4 hours are the same as the preferred range, measurement method, and control method for the weight loss rate when the higher order structure of the first embodiment described above is immersed in phosphate buffered saline at 50°C for 4 hours.

The fiber sheet structure of the present invention comes to exhibit tactility in a wet state similar to that of biological tissue by containing gelatin fiber bundles, and thus is characterized by exhibiting more slipperiness compared with a fiber sheet structure made only of thermoplastic resin fiber bundles. Generally, a fiber sheet structure made of a thermoplastic resin fiber bundle has a problem of great difficulty due to the hydrophobicity in impregnating with a cell culture medium. However, the fiber sheet structure of the present invention contains gelatin fiber bundles with excellent hydrophilicity, thus enables not only cell culture fluid to easily impregnate and to be retained, but also body fluids containing oxygen and nutrients to spread within the fiber sheet structure, and as a result, cells to easily colonize and proliferate inside the fiber sheet structure.

A method for producing the fiber sheet structure of the present invention will be described below though not particularly limited thereto.

The method for producing the fiber sheet structure of the present invention preferably includes the following steps 1 and 2, and step 3-1, step 3-2, or step 3-3, and step 4-1 or step 4-2.

(Step 1) A step of extruding a dope, made of a gelatin solution containing 30 to 70 wt% of a gelatin raw material, from a multi-hole spinneret into a fiber form, at a temperature 1 to 15°C higher than the sol-gel transition temperature of the gelatin dope;

(Step 2) A step of winding the extruded gelatin fiber around a bobbin to prepare a gelatin fiber bundle precursor;

(Step 3-1) A step of doubling the gelatin fiber bundle precursor with a thermoplastic resin fiber, and then weaving it to prepare a second higher order structure;

(Step 3-2) A step of doubling the gelatin fiber bundle precursor with a thermoplastic resin fiber, weaving it, and then laminating a gelatin nonwoven fabric thereon to produce a second laminated higher order structure;

(Step 3-3) A step of insolubilizing the gelatin fiber bundle precursor, doubling it with a thermoplastic resin fiber, and further weaving it to prepare a second higher order structure;

(Step 4-1) A step of insolubilizing the gelatin contained in the second higher order structure obtained in Steps 3-1 or the second laminated higher order structure obtained in 3-2 and then pressing them to prepare a fiber sheet structure;

(Step 4-2) A step of pressing the second higher order structure obtained in Step 3-3 to produce a fiber sheet structure;

The step 2 includes a conveying step of conveying the extruded gelatin fiber along the peripheral surface of a conveying roller before being wound around a bobbin, wherein the extruded gelatin fiber preferably has a gelatin fiber moisture content of 5 to 25 wt% on arriving at the first conveying roller at which the extruded gelatin fiber arrives first.

The moisture content of the gelatin fiber bundle precursor is preferably 5 to 20 wt%.

The step 2 includes a step of applying a mixed solution of at least one selected from ethylene glycol, polyethylene glycol, and glycerin and a lower alcohol to the extruded gelatin fibers before being wound around a bobbin.

In the step 1, preferably, the gelatin has a jelly strength of 100 to 350 g, and the gelatin dope has a sol-gel transition temperature of 15 to 60°C.

The fiber sheet structure of the present invention can be produced by extruding fibrously a dope made of a gelatin solution from a multi-hole spinneret, winding the extruded gelatin fibers around a bobbin to produce a gelatin fiber bundle precursor, doubling the gelatin fiber bundle precursor with a thermoplastic resin fiber and then weaving the resulting bundle to obtain a second higher order structure, or laminating a nonwoven fabric on the second higher order structure to produce a second laminated higher-order structure, either of which is insolubilized and then pressed to produce a fiber sheet structure. As described above, a fiber sheet structure may also be produced by doubling a gelatin fiber bundle precursor insolubilized in advance with a thermoplastic resin fiber, and then weaving it to obtain a second higher order structure, and pressing the resulting second higher order structure.

Note that, the term "weaving" refers to the production of a woven fabric, knitted fabric, or braided cord from doubled yarn bundles.

The gelatin raw material constituting the gelatin solution in the fiber sheet structure of the present invention is obtained as a single molecular chain by unraveling the triple helix of collagen derived from cow bones, cowhide, pigskin, birds, and fish. Methods for producing such a gelatin raw material include an acid treatment method and a lime treatment method, and the gelatin raw material in the fiber sheet structure of the present invention may be a gelatin raw material produced by either method, or may be a commercially available gelatin raw material. In addition, commercially available gelatin raw materials undergo various purification steps before being extracted in the manufacturing process, thus contain few components other than proteins, and generally have a composition of 85% or more protein, 8 to 14% water, 2% or less ash, and 1% or less other components (lipids, polysaccharides, etc.), and such general gelatin raw materials can also be used in the present invention. Although gelatin raw materials have an amino acid composition almost the same as that of collagen because of a heat-denatured material of collagen, the composition is highly specific compared to other proteins: glycine accounts for about one-third of the total and is repeated as one in every three in the amino acid sequence. Collagen and gelatin can be distinguished from each other, for example, by observing a signal around 31.5 degrees obtained by wide-angle X-ray measurement. This signal at 31.5 degrees is a signal derived from the triple helical structure of collagen, and when the triple helical structure characterizing collagen is unraveled to become gelatin, this signal disappears.

In addition, although the molecular weight of the gelatin raw material is not particularly limited, the gelatin raw material has a jelly strength, which is highly correlated with the molecular weight, of preferably 100 to 350 g, more preferably 150 to 320 g, and further preferably 220 to 310 g, from the viewpoint of spinnability. The jelly strength is a load (g) required to press down 4 mm the surface of the gelatin solution prepared by cooling a 6.67% gelatin solution at 10°C for 17 hours, with a plunger having a diameter of 1/2 inch (12.7 mm), using a small tabletop tester EZ-SX manufactured by Shimadzu Corporation.

The jelly strength of the gelatin raw material within the above range can prevent a significant decrease in the spinnability of the gelatin fiber, and further suppressing the moisture retention rate in the fiber can prevent breakage of the fiber during winding.

The content of the gelatin raw material in the gelatin solution is preferably 30 to 70 wt%, and more preferably 40 to 60 wt%. The content of the gelatin raw material in the gelatin solution within the above range can prevent a significant decrease in the spinnability of the gelatin fiber, and further can facilitate degassing bubbles generated during the gelatin solution production process, thereby preventing yarn breakage during spinning.

The solvent for dissolving the gelatin raw material is water, and (polyhydric) alcohols, dimethyl sulfoxide, etc. may be added to adjust the viscosity, if necessary.

The sol-gel transition temperature of the gelatin solution is preferably 15 to 60°C, more preferably 20 to 60°C, further preferably 20 to 55°C, and particularly preferably 20 to 50°C. The sol-gel transition temperature is measured, for example, by using a rheometer ARES manufactured by TA Instruments.

The sol-gel transition temperature within the above range can prevent increase in the adhesiveness, thus improve the handleability during the production of the gelatin fiber, and further, prevent adhesion of the wound gelatin fiber. In addition, can be prevented the thermal decomposition of gelatin at the spinning temperature, and possible occurrence of yarn breakage.

The gelatin fiber bundle precursor is produced by extruding fibrously a dope, made of gelatin raw material and a solvent such as water, from a multi-hole spinneret into the air, and by moderately orienting the molecular chains of the triple helix molecules of collagen loosened by drawing just below the spinneret, while moderately controlling the moisture content in the fibers. Controlling the degree of orientation of the molecular chains of the loosened collagen triple helix molecules and the moisture content in the fibers allow the tensile strength and breaking elongation of the yarn to increase, and at the same time, enables suppression of adhesion between the fibers.

The spinning method of extruding fibrously the dope into the air from a multi-hole spinneret may be any of a dry method, a semi-dry method, and a wet method, among which the dry method is preferable for preventing the formation of voids on the surface and inside of the fiber during the process of drying and removing the solvent and thus suppressing reduction of the tensile strength and breaking elongation of the gelatin fiber. The dry spinning temperature is preferably 1 to 25°C higher than the sol-gel transition temperature of the dope, and more preferably 5 to 20°C higher. Within the above range, a significant decrease in spinnability can be prevented.

In the production of the gelatin fiber bundle precursor used in the present invention, the moisture content in the fibers is preferably reduced rapidly just after spinning to a range of 5 to 25 wt%. The moisture content in the fiber within the above range enables suppression of the stickiness, and prevention of adhesion among the gelatin fibers discharged from the multi-hole spinneret and sticking of the gelatin fibers to rollers during the producing step, and the gelatin fiber precursor wound around the bobbin to be easily unwound from the bobbin. Furthermore, enabled are suppression of significant decrease in the elongation of the yarn, and thus prevention of yarn breakage during the step of producing gelatin fiber bundles and during the process of producing a fiber sheet structure while rewinding the bundle from the bobbin.

There may be included a conveying step of conveying the gelatin fibers extruded from the spinneret along the peripheral surface of a conveying roller before winding the gelatin fibers around a bobbin.

The moisture content of the gelatin fiber is preferably 5 to 25 wt%, and more preferably 5 to 20 wt% on arriving at the first conveying roller at which the extruded gelatin fiber arrives first. The above moisture content is obtained by winding a gelatin yarn around a first conveying roller and measuring 0.5 g of the wound fiber at 150°C in an N2 atmosphere using a Karl Fischer moisture content meter (EV-2010 manufactured by HIRANUMA Co., Ltd.).

The moisture content within the above range enables prevention of sticking of the gelatin fibers to the conveying rollers, and suppression of a significant decrease in the elongation of the yarn, and thus prevention of yarn breakage.

The moisture content can be controlled by drying with dry air or dry hot air.

In order to prevent adhesion among the fibers due to contact, grooved guide rollers are preferably used as the conveying rollers.

The moisture content of the gelatin fiber bundle precursor of gelatin fibers wound around a bobbin is preferably 5 to 20 wt%, and more preferably 5 to 15 wt%. The above moisture content is obtained by measuring 0.5 g of the fiber bundle precursor wound around a bobbin at 150°C in an N2 atmosphere using a Karl Fischer moisture content meter (EV-2010 manufactured by HIRANUMA Co., Ltd.).

The moisture content within the above range enables the gelatin fiber precursor to be easily unwound from the bobbin, and suppression of a significant decrease in the elongation of the yarn, and thus prevention of breakage of the yarn during the process of producing a fiber sheet structure while rewinding the yarn from the bobbin.

The moisture content can be controlled by drying with dry hot air or the like. In addition, after passing through the first conveying roller, the fiber bundle can be dried timely by a winder.

The gelatin fiber bundle used in the present invention is preferably produced at a spinning draft (discharge linear speed/winding speed) in the range of 0.01 to 2, and more preferably 0.05 to 1.5. The value within the above range enables the loosened collagen triple helix molecules to have sufficient orientation to prevent a decrease in tensile strength, and the fiber diameter to be prevented from becoming too thick, and the moisture content of the gelatin fibers to be sufficiently reduced before arriving at the first conveying roller, and as a result, the gelatin fibers to be prevented from sticking to the roller. Furthermore, enabled are suppression of significant decrease in the breaking elongation, and thus prevention of yarn breakage during the step of producing gelatin fiber bundles and during the process of producing a fiber sheet structure while rewinding the bundle from the bobbin.

There may be included a step of applying an oily agent to the gelatin fibers extruded from the spinneret before being wound around a bobbin. The surface of the fibers is applied with an oily agent preferably after the moisture content in the fibers reaches the range of 5 to 20 wt%. Applying an oily agent to the fiber surface enables multiple gelatin fibers to be bundled together and handled as a gelatin fiber bundle. The application of the oily agent significantly improves the handleability of the yarn during the step of producing gelatin fiber bundles and during the process of producing a fiber sheet structure while rewinding the yarn from the bobbin. Since the fiber sheet structure of the present invention is used as a medical material, a biocompatible oily agent is preferably used, such as ethylene glycol, polyethylene glycol, or glycerin. These compounds are preferably used by diluting with a lower alcohol such as ethanol, and applied as a mixed solution to the fiber surface, and then the lower alcohol is removed by drying. The oily agent may be removed by washing after the production of the fiber sheet structure, or may be left as applied. The amount of oily agent applied has no restriction as long as allowing a plurality of gelatin fibers to be bundled into a fiber bundle, and is preferably, for example, 0.5 to 5 wt%.

The cross-sectional shape of the gelatin fibers may be any shape. Examples of fiber cross-sectional shapes include solid circle, hollow, flat, star, triangle, solid circle+fin, hollow+fin. When used as a three-dimensional cell culture scaffold material, irregular cross-section yarns having irregular cross-sections such as flat, star, or triangle may be preferable to solid circle.

The tensile strength of the gelatin fiber is preferably 0.5 to 3 cN/dtex, more preferably 0.7 to 2.8 cN/dtex, and further preferably 1.0 to 2.5 cN/dtex. The tensile strength of the gelatin fiber is measured by taking out a single yarn from the gelatin fiber bundle precursor and using, for example, Tensilon (RTC-1210A manufactured by Orientec Co., Ltd.).

The breaking elongation of the gelatin fiber is preferably 30 to 250%, more preferably 40 to 200%, and further preferably 50 to 150%. The breaking elongation of the gelatin fiber is measured by taking out a single yarn from a gelatin fiber bundle and using, for example, Tensilon (RTC-1210A manufactured by Orientec Co., Ltd.).

The tensile strength and breaking elongation of the gelatin fiber within the above ranges enable the gelatin fiber to withstand the production of gelatin fiber bundles and the production of fiber sheet structure.

The step of insolubilizing the gelatin fiber bundle precursor may be either of insolubilizing the gelatin fiber bundle precursor to produce gelatin fiber bundles, or doubling the fiber bundle precursor and thermoplastic resin fiber and weaving the doubling bundles, and then insolubilizing to produce a fiber sheet structure made of gelatin fiber bundles. The insolubilization (treatment to make the material insoluble in water) may be performed by any of the following methods: thermal crosslinking, chemical crosslinking using a crosslinking agent, and physical crosslinking using ultraviolet light, radiation, or electron beams, or a combination of these methods may be used as appropriate. When thermal crosslinking is performed, it may be performed in air or under vacuum. The temperature during thermal crosslinking is preferably in the range of 110 to 200°C, more preferably 120 to 190°C, and further preferably 130 to 180°C. If the temperature is lower than 100°C, crosslinking will be insufficient, and the gelatin will dissolve in water in a wet state, making it unusable as a scaffold material. If the temperature is higher than 200°C, the gelatin will thermally decompose, causing a significant decrease in fiber strength, which is not preferable. The degree of crosslinking can be changed timely by the combination of the heat treatment temperature and the heat treatment time, which is preferably 3 to 72 hours and from the viewpoint of productivity, is preferably 12 to 50 hours. Chemical crosslinking using a crosslinking agent may induce formation of pores in the fibers during the washing process to remove the crosslinking agent in the fibers, which may reduce the tensile strength and breaking elongation of the gelatin fibers. In this case, yarn breakage may occur during the process of preparing a fiber sheet structure from the insolubilized gelatin fiber bundles. For this reason, the insolubilization treatment by chemical crosslinking is preferably performed after weaving the fiber bundle precursor. Examples of crosslinking agents used in chemical crosslinking include glutaraldehyde and hexamethylene diisocyanate.

The tensile strength and breaking elongation of insolubilized gelatin fibers are measured by pulling out a single yarn from a gelatin fiber bundle obtained by insolubilizing a gelatin fiber bundle precursor, and measuring the single yarn using, for example, Tensilon (RTC-1210A manufactured by Orientec Co., Ltd.). The breaking elongation of the insolubilized single yarn is preferably 30 to 250%, more preferably 40 to 200%, and further preferably 50 to 150%. The tensile strength and breaking elongation of the insolubilized single yarn within the above ranges enable the fiber sheet structure to withstand the production of fiber sheet structure.

The thermoplastic resin fiber in the fiber sheet structure of the present invention can be produced by melt spinning, and a commercially available thermoplastic resin fiber bundle can also be purchased for use.

The doubled yarn bundle in the fiber sheet structure of the present invention can be produced using a starting material of a doubled yarn bundle precursor made of a gelatin fiber bundle precursor and a thermoplastic resin fiber bundle. A doubled yarn bundle precursor made of a gelatin fiber bundle precursor and a thermoplastic resin fiber bundle can be produced from a plurality of fiber bundle precursors using a doubling machine.

Twisting may be performed by spirally winding the thermoplastic resin fiber bundle around the gelatin fiber bundle precursor, or by spirally winding the gelatin fiber bundle precursor around the thermoplastic resin fiber bundle.

Doubling may be performed by twisting the gelatin fiber bundle precursor and the thermoplastic resin fiber bundle both with oily agent as applied thereto, or by twisting the fiber bundles with each other by interlacing etc.

In addition, gelatin fiber bundle precursors set in a plurality of bobbins and thermoplastic resin fiber bundles set in a plurality of bobbins in a knitting machine may be doubled during conveying to the knitting needle of the knitting machine.

The doubling yarn composed of the gelatin fiber bundle precursor and the thermoplastic resin fiber bundle may be composed of the gelatin fiber bundle insolubilized in advance and the thermoplastic resin fiber bundle. The doubled yarn bundle is produced, and then can be woven to produce a second higher order structure. The second high-order structural body may be laminated with a nonwoven fabric to form a second laminated higher order structure. For example, in the case of laminating a gelatin or collagen nonwoven fabric, the following method can be exemplified: a second higher order structure is woven, on which a nonwoven fabric is laminated by electrospinning or the like. The laminated gelatin or collagen nonwoven fabric can be water-insolubilized by a crosslinking treatment.

The second higher order structure can be produced, for example, by using WHOLEGARMENT (registered trademark) manufactured by Shimadzu Corporation. The gauge is preferably 5 to 20. The gauge less than 5 requires the fineness of the doubled yarn bundle to be high, which will clog the mesh of the second higher order structure to inhibit cell penetration into the fiber sheet structure from the cell-mounting front surface; In contrast, the gauge more than 20 requires the fineness of the doubled yarn bundle to be low, which will result in weak strength of the doubled yarn bundle and thus may cause yarn breakage during production of the second higher order structure. For this reason, the gauge is more preferably 10 to 20, and most preferably 13 to 18.

The second higher order structure may be a single-knit fabric consisting of one layer (sheet) or may be a double-knit fabric consisting of two laminated layers. The double-knit fabric having two laminated layers can be produced by knitting two layers separately using, for example, WHOLEGARMENT (registered trademark) manufactured by Shimadzu Corporation while knitting the two layers together.

The knitted fabric of the second higher order structure is preferably jersey stitch or plain stitch, and rib stitch. The jersey stitch or plain stitch has highly flat and smooth surface due to the minimal unevenness of the fabric surface, and thus exhibit a slipperiness similar to that of living tissue. In contrast, the sheet structure curls to form a cylindrical sheet structure, and thus will cause the fiber sheet structure to have not only difficulty in colonizing the cells uniformly but also poor handleability. For this reason, the two layers preferably form a two-layer laminated structure to suppress curling by knitting together two sheet layers, upper one of jersey stitch or plain stitch curling convexly upwards and the lower one of jersey stitch or plain stitch curling convexly downwards, to make the stress acting on both layers about the same. Furthermore, laminating two layers will allow the area for colonizing cells to increase and the frequency of cell slipping-off through the mesh to be significantly reduced, as a result, a fiber sheet structure to exhibit excellent cell cultivatability. Furthermore, laminating a nonwoven fabric, having an average pore size overwhelmingly smaller than that of a knitted fabric enables cells having penetrated from the cell-mounting front surface side to be retained more within the fiber sheet structure, and as a result, a fiber sheet structure to exhibit excellent cell cultivatability.

Rib stitch, though not curling due to the same weave on the front and back surface, has the fabric surface more uneven than jersey stitch or plain stitch. However, the pressing process used during the fabric sheet structure production can eliminate the unevenness on the fabric surface, allowing the surface to exhibit a smooth surface nature and slipperiness similar to that of living tissue. The rib stitch does not curl, allowing cells to colonize uniformly in the fiber sheet structure. Although not requiring two layers for this reason, the fiber sheet structure can also be laminated with a nonwoven fabric having average pore size overwhelmingly smaller than that of knitted fabric to retain within the fiber sheet structure more cells having penetrated from the cell-mounting front surface side. Furthermore, compared to a fiber sheet structure laminating two layers of knit weave, the fiber sheet structure can have thinner thickness due to the single layer knit weave, and thus exhibit a softer texture.

The second higher order structure used in producing the fiber sheet structure preferably has an average pore size of 20 to 200 $\mu$m. The fiber sheet structure is produced by pressing the second higher order structure or the second laminated higher order structure (nonwoven fabric surface) while applying a temperature of 40 to 100°C to one side. The press treatment crushes the mesh on both the front and back surfaces of the second higher order structure, and more heavily on the surface heated to 40 to 100°C than on the surface not heated, resulting in an asymmetric fiber sheet structure with a back surface having a smaller average pore size than the cell-mounting front surface. The second higher order structure with average pore size of 20 to 200 $\mu$m can be subjected to the press treatment to obtain a fiber sheet structure having excellent cell cultivatability, having the average pore size of 5 to 123 $\mu$m on the cell-mounting front surface and the proportion of 35% or less for the number of pores with a size of 150 $\mu$m or more on the cell-mounting front surface. When the average pore size of the second higher order structure is less than 20 $\mu$m, the mesh of the fiber sheet structure will be blocked by the press treatment, thereby inhibiting cell penetration from the cell-mounting front surface into the inside of the fiber sheet structure, and when the average pore size of the second higher order structure is more than 200 $\mu$m, the mesh cannot be sufficiently crushed by the press treatment, causing the cells to slip off through the mesh of the fiber sheet structure. The average pore size of the second higher order structure is preferably 25 to 150 $\mu$m, and more preferably 30 to 130 $\mu$m.

The fiber sheet structure of the present invention has a cell-mounting front surface and a back surface, the average pore size of the back surface is smaller than the average pore size of the cell-mounting front surface, and thus both surfaces are asymmetrical. Such a fiber sheet structure having asymmetric surfaces is produced, for example, by cleaning off oily agent or knitting machine oil adhering to the second higher order structure or the second laminated higher order structure, and then pressing while applying a temperature of 40 to 100°C to one side of the second higher order structure or the nonwoven fabric side of the second laminated higher order structure. Although the reason is not clear, it is probably because the binding of the fiber bundles was reduced by removing the oily agent and knitting machine oil, the fiber bundles were unraveled further due to the difference in thermal linear expansion between the gelatin fiber and the thermoplastic resin fiber, and the fiber bundles were then expanded by the press. Use of this method enables production of a fiber sheet structure having a cell-mounting front surface and a back surface, with the average pore size on the back surface smaller than the average pore size on the cell-mounting front surface, and with both surfaces asymmetric. Examples of a device for pressing the fiber sheet structure by applying heat to only one side thereof include a temperature-modulatable hydraulic press and a calender roll.

The fibrous sheet structure produced in the present invention may be immersed in, for example, a 20 to 80% glycerin aqueous solution. Immersing in an aqueous glycerin solution allows the fiber sheet structure to be produced as a medical material having better followability to the affected area and a moist texture.

The fiber sheet structure of the present invention may contain an additive. Examples of the additive include polyacids such as (anhydrous) citric acid and (anhydrous) maleic acid, magnetic particles such as gamma-ferric oxide, inorganic substances such as hydroxyapatite, endogenous proteins that promote cell proliferation and differentiation, and collagen. These additives may be applied on the fiber sheet structure, or may be applied on the gelatin fiber bundles, gelatin fiber bundle precursors, or gelatin fibers, or may be added to the gelatin raw material.

<Characteristics of fiber sheet structure>

The fiber sheet structure of the present invention is excellent in texture, slipperiness, cell adhesiveness, and cell cultivatability.

The texture of the fiber sheet structure of the present invention can be evaluated based on the touch feeling when the fiber sheet structure is touched with dry hands. The texture was evaluated by three-time repetition of pinching the fiber sheet structure cut into a 5 cm square at both ends with both hands and pulling them, and by pulling one pinched end upwards and the other end downwards. No stretchability is likely to cause a stiff texture, which makes it difficult to follow biological tissue, and thus a moderate stretchability is required.

The slipperiness of the fiber sheet structure of the present invention can be evaluated using a friction tester (model number: KES-SE) manufactured by Kato Tech Co., Ltd. The average friction coefficient and the average deviation of the friction coefficient were measured by immersing in distilled water for 1 hour a sample of the fiber sheet structure cut into 5 cm square, then fixing the sample on a measuring table with a metal frame, and running a 10 mm square silicon sensor over the sample at 1.0 mm/sec with a load of 50 g.

The average friction coefficient of the fiber sheet structure of the present invention is preferably 0.05 to 0.60, more preferably 0.10 to 0.50, further preferably 0.15 to 0.45, and most preferably 0.20 to 0.40. Within the above range, the slipperiness is similar to that of living tissue, and inflammation is unlikely to occur on rubbing against living tissue.

The average deviation of the friction coefficient of the fiber sheet structure of the present invention is preferably 0.40 to 1.60, more preferably 0.45 to 1.45, further preferably 0.50 to 1.40, and most preferably 0.55 to 1.35. Within the above range, the fiber sheet structure of the present invention has softness and slipperiness similar to those of living tissue under wet conditions. For this reason, these can be suitably used as a material for treating wound/burn, a matrix material for artificial skin, etc., and are unlikely to cause inflammation on rubbing against living tissue, which is preferable.

The fiber sheet structure of the present invention has excellent cell adhesiveness for colonizing cells. Cell adhesiveness strongly correlates with cell cultivatability: strong cell adhesiveness allows cells to efficiently colonize in the fiber sheet structure to exert excellent cell cultivatability. In contrast, low cell adhesiveness cannot allow cells to colonize well in the fiber sheet structure, which tends to exert poor cell cultivatability. Cell cultivatability can be evaluated by the number of cultured cells obtained by statically culturing NIH3T3 cells for 7 days on the fiber sheet structure placed on a 24-well plate, adding 100 $\mu$L of WST-1, leaving the cells in a $CO_2$ incubator (37°C, $CO_2$ concentration 5%) for 3 hours, then transferring 300 $\mu$L of the cells to a 96-well plate to measure the absorbance using a plate reader (VERSA Max) (450 nm, reference wavelength 630 nm) for determining the number of cultured cells. The number of cultured cells is preferably $10 \times 10^4$ cells/mL or more, more preferably $20 \times 10^4$ cells/mL or more, and further preferably $25 \times 10^4$ cells/mL or more.

Gelatin is a biocompatible material (low antigenicity and high bioabsorbability), and therefore can be suitably used as a scaffold material for three-dimensional cell culture, which is excellent in promoting cell differentiation, enhancing cell function, and colonizing after transplantation. Furthermore, the fiber sheet structure of the present invention in a wet state has softness and slipperiness similar to those of living tissue. For this reason, these can be suitably used as a material for treating wound/burn, a matrix material for artificial skin, etc., and are unlikely to cause inflammation on rubbing against living tissue, which is preferable.

The cell adhesiveness of the fiber sheet structure can be evaluated by statically culturing NIHT3T3 cells on the structure for 7 days and observing the fluorescently labeled cells under a fluorescence microscope to determine the degree of adhesiveness of the cells.

The none-curling fiber sheet structure of the present invention not only enables cells to colonize uniformly inside, but also exhibit excellent handleability. The presence or absence of curling was measured by visually checking the sample prepared by punching the fiber sheet structure with a hole punch to prepare a sample of circular sheet with a diameter of 14 mm, immersing the sample in a beaker containing phosphate buffered saline at 50°C for 4 hours.

[Examples]

The fiber sheet structure of the present invention will be described more specifically below with reference to examples. The materials, amounts used, ratios, processing procedures, and the like shown in the following examples can be changed as appropriate as far as not departing from the spirit of the present invention. Therefore, the scope of the fiber sheet structure of the present invention should not be construed as being limited by the specific examples shown below.

<Measurement method and evaluation method>

The measurement and evaluation methods used in the examples and comparative examples are as follows.

[Fiber fineness]

Using an auto vibro type fineness measuring instrument Denier Computer DC-77A (manufactured by Search Co., Ltd.), the fineness of the thermoplastic resin fiber bundle was measured in an atmosphere of a temperature of 20 ± 2°C and a relative humidity of 65 ± 2%, with N=3, and the average value was taken as the fineness. The fineness of the thermoplastic resin fiber was calculated by dividing the fineness of the thermoplastic resin fiber bundle by the number of thermoplastic resin fibers constituting the fiber bundle.
The gelatin fiber bundle precursor or the gelatin fiber bundle was cut to 100 mm, and the weight was measured with an electronic balance (XP6V manufactured by Mettler Toledo) for N=3, and the average value was converted into dtex to obtain the fineness. The fineness of the gelatin fiber was calculated by dividing the fineness of the gelatin fiber bundle precursor or the fineness of the gelatin fiber bundle by the number of gelatin fibers constituting the fiber bundle.

[Mechanical strength of fiber]

The mechanical strength of the gelatin fibers and the insolubilized gelatin fibers was measured using Tensilon (RTC-1210A manufactured by Orientec Co., Ltd.) with N=5. Note that, the pulling speed was 20 mm/min, and the distance between chucks was 50 mm, and the tensile strength and breaking elongation of the gelatin fiber were measured.

[Mechanical strength of fiber bundle and doubled yarn bundle]

The mechanical strength of the gelatin fiber bundle or the doubled yarn bundle was measured using a Tensilon (RTC-1210A manufactured by Orientec Co., Ltd.) with N=5. Note that, the pulling speed was 20 mm/min, the distance between chucks was 50 mm, the tensile strength and breaking elongation of the gelatin fiber bundle or doubled yarn bundle, and the upper yield stress and lower yield stress of the gelatin fiber bundle were measured.

[Weight loss rate of gelatin fiber bundle and doubled yarn bundle]

The weight of a 100 mm gelatin fiber bundle or doubled yarn bundle was measured, and the bundle was wrapped in a 10 μm membrane filter, and immersed in phosphate buffered saline at 50°C for 4 hours. Next, the membrane filter containing the gelatin fiber bundle or the gelatin doubled yarn bundle was pulled up from the phosphate buffered saline and immersed in water at room temperature for 30 minutes. Thereafter, the moisture was absorbed with a Kimtowel, and the membrane filter containing the gelatin fiber bundle or the doubled yarn bundle was dried at 80°C for 24 hours, and then the fiber bundle was taken out from the membrane filter and was measured for the weight. The weight loss rate was calculated using the following formula. This operation was carried out with N=3, and the average value was taken as the weight loss rate.

Weight loss rate [%] = 100 - [100 × (weight after immersion)] / (weight before --> immersion)

[Weight loss rate of higher order structure, laminated higher order structure, and fiber sheet structure]

The weight of 25 cm$^2$ higher order structure, laminated higher order structure or fiber sheet structure was measured, the structure was wrapped in a 10 μm membrane filter, immersed in phosphate-buffered saline at 50°C for 4 hours, the membrane filter containing the higher order structure, laminated higher order structure or fiber sheet structure was then pulled up from the phosphate-buffered saline, immersed in water at room temperature for 30 minutes, the water was then absorbed with a Kimtowel, the membrane filter containing the higher order structure, laminated higher order structure and fiber sheet structure was dried at 80°C for 24 hours, and the higher order structure, laminated higher order structure or fiber sheet structure was then taken out from the membrane filter, and was measured for the weight. The weight loss rate was calculated using the following formula. This operation was carried out with N=3, and the average value was taken as the weight loss rate.

Weight loss rate [%] = 100 - [100 × (weight after immersion)] / (weight before immersion)

[Thickness of higher order structure, laminated higher order structure, and fiber sheet structure]

Adhesive tape was applied to the end of the higher order structure, laminated higher order structure, or fiber sheet structure cut into a 3 cm square to fix the higher order structure, laminated higher order structure, or fiber sheet structure, and the thickness was measured using a contact type thickness meter (LITEMATIC VL-50, manufactured by Mitutoyo Corporation). A cylindrical terminal having a diameter of 5 mm was used as the measurement terminal, and was adjusted so that a load of 7 g was applied during measurement. The thickness of the higher order structure, the laminated higher order structure, or the fiber sheet structure was measured at arbitrary five points, and the average value was taken as the thickness of the higher order structure, the laminated higher order structure, or the fiber sheet structure.

[Average pore size of higher order structure, laminated higher order structure and fiber sheet structure]

The average pore size on the cell-mounting front surface was determined as follows: obtaining a 35× image (pixel count: 1920 × 1200) of a higher order structure, laminated higher order structure or fiber sheet structure cut into a 5 cm square using a digital microscope (RH-2000, manufactured by Hirox Co., Ltd.), then binarizing the obtained image into pores and non-pores using the 2D measurement software "HRS-2D" attached to the microscope, using the same software to create a histogram of pore sizes at 10 μm intervals from 60 to 300 μm, and calculating the average pore size in accordance with the following formula using the class value and frequency obtained from this histogram.

$$\text{Average pore size (μm)} = \{\Sigma \,(\text{class value} \times \text{frequency})\} \div (\Sigma \,\text{frequency})$$

[Proportion of number of pores with a size of 150 μm or more on cell-mounting front surface of higher order structure, laminated higher order structure, and fiber sheet structure]

The proportion of number of pores with a size of 150 μm or more on the cell-mounting front surface of the higher order structure, laminated higher order structure, or fiber sheet structure was calculated using the histogram used to calculate the average pore size described above, by dividing the total frequency of pores with a size of 150 μm or more by the total frequency of pores with sizes of 60 to 300 μm.

[Porosity of higher order structure, laminated higher order structure, and fiber sheet structure]

The porosity was calculated using the following formula by measuring the thickness and weight of the higher order structure, laminated higher order structure, or fiber sheet structure cut into a 5 cm square.

$$\text{Porosity (\%)} = 100 \times [1 - a \div \{(b + c) \div (b \div d + c \div e)\} \div (f \times 25)]$$

a: Weight (g) of higher order structure, laminated higher order structure, or fiber sheet structure cut into a 5 cm square
b: Gelatin fiber bundle fineness (dtex)
c: Thermoplastic fiber bundle fineness (dtex)
d: Gelatin fiber bundle density (g/cm$^3$)
e: Thermoplastic fiber bundle density (g/cm$^3$)
f: Thickness (cm) of higher order structure, laminated higher order structure, or fiber sheet structure

Note that, the following values are used for each density: gelatin fiber bundle density of 1.27 g/cm$^3$, thermoplastic fiber bundle density including polyglycolic acid fiber bundle density of 1.53 g/cm$^3$ and polylactic acid fiber bundle density of 1.25 g/cm$^3$. In addition, when the higher order structure is produced using only gelatin fiber bundles without using thermoplastic fiber bundles, the above c and e are not included in the calculation.

[Number of pores in higher order structure, laminated higher order structure and fiber sheet structure]

The number of pores in the higher order structure, the laminated higher order structure, or the fiber sheet structure was calculated by the following formula using the average pore size and porosity obtained above.

$$\text{Number of holes/mm}^3 = a \times 0.01 \div (4/3 \times 3.14 \times (0.001 \times b/2)^3)$$

a: Porosity (%)
b: Pore size ($\mu$m)

[Swelling ratio of gelatin fiber bundle]

The fiber diameter of the gelatin fibers constituting the gelatin fiber bundle was measured using an optical microscope RH2000 manufactured by HIROX Co., Ltd. Next, 10 to 30 mm of the gelatin fiber bundle was immersed in phosphate buffered saline at 50°C for 4 hours, and then the gelatin fiber bundle was pulled up from the phosphate buffered saline, and the fiber diameter of the gelatin fibers constituting the gelatin fiber bundle in a wet state was measured. The swelling ratio was calculated using the following formula. This operation was carried with N=3, and the average value was taken as the swelling ratio.

Swelling ratio [%] = 100 $\times$ (fiber diameter after immersion) / (fiber diameter before immersion)

[Swelling ratio of doubled yarn bundle]

The bundle diameter of the doubled yarn bundle was measured using an optical microscope RH2000 manufactured by HIROX Co., Ltd. Next, 10 to 30 mm of the doubled yarn bundle was immersed in phosphate buffered saline at 50°C for 4 hours, and then the doubled yarn bundle was pulled up from the phosphate buffered saline, and the fiber diameter of the doubled yarn in a wet state was measured. The swelling ratio was calculated using the following formula. This operation was carried with N=3, and the average value was taken as the swelling ratio.

Swelling ratio [%] = 100 $\times$ (diameter of doubled yarn bundle after immersion) / (diameter of doubled yarn bundle before immersion)

[Moisture content of fiber bundle precursor]

The moisture content is obtained by measuring 0.5 g of the fiber bundle precursor wound around a bobbin at 150°C in an N2 atmosphere using a Karl Fischer moisture content meter (EV-2010 manufactured by HIRANUMA Co., Ltd.).

[Moisture content of gelatin fiber on arriving at conveying roller]

The moisture content is obtained by winding a gelatin yarn around a first conveying roller and measuring 0.5 g of the wound fiber at 150°C in an N2 atmosphere using a Karl Fischer moisture content meter (EV-2010 manufactured by HIRANUMA Co., Ltd.).

[Jelly strength]

A 6.67% gelatin aqueous solution was cooled at 10°C for 17 hours. Next, the jelly strength was taken as a load (g) required to press down 4 mm the surface of the gelatin solution with a plunger having a diameter of 1/2 inch (12.7 mm), using a small tabletop tester EZ-SX manufactured by Shimadzu Corporation.

[Sol-gel transition temperature]

The temperature dependence of viscoelasticity was measured on the gelatin dope, when temperature was raised at 1 K/min in the temperature range of 20 to 70°C, using a rheometer ARES manufactured by TA Instruments, and the temperature at which the magnitude relationship between the storage elastic modulus and the loss elastic modulus was exchanged was determined as the sol-gel transition temperature.

[Texture of higher order structure, laminated higher order structure, and fiber sheet structure]

The texture was evaluated based on a sensory evaluation of the touch feeling when the higher order structure, the laminated higher order structure, or fiber sheet structure was touched with dry hands.

Excellent: Stretchable and soft texture
Good: The obtained structure having lost stretchability, but stretchability and moist texture regained by immersing in a 60% glycerin aqueous solution
Average: Stretchable but rough texture
Poor: Structure improducible and hard texture

[Average friction coefficient and average deviation of friction coefficient of higher order structure, laminated higher order structure, and fiber sheet structure]

The average friction coefficient and the average deviation of the friction coefficient of the higher order structure, the laminated higher order structure, or the fiber sheet structure were evaluated using a friction tester (model number: KES-SE) manufactured by Kato Tech Co., Ltd. The average friction coefficient and the average deviation of the friction coefficient were measured by immersing in distilled water for 1 hour a sample of the higher order structure, the laminated higher order structure, or fiber sheet structure cut into 5 cm square, then fixing the sample on a measuring table with a metal frame, and running a 10 mm square silicon sensor over the sample at 1.0 mm/sec with a load of 50 g. After performing this operation for N=3, the sample was rotated by 90° and the same operation was repeated, and the span of the surface friction coefficient (minimum and maximum values) and the span of the average deviation of the friction coefficient (minimum and maximum values) were determined as measurement data.

[Measurement of curl of higher order structure, laminated higher order structure, and fiber sheet structure]

The presence or absence of curling was evaluated by visually checking the sample prepared by punching the higher order structure, the laminated higher order structure, or the fiber sheet structure with a hole punch to prepare a sample of circular sheet with a diameter of 14 mm, immersing the sample in a beaker containing phosphate buffered saline at 50°C for 4 hours.

[Evaluation of number of cultured cells in fiber sheet structure]

Cell culture in the higher order structure, the laminated higher order structure, and the fiber sheet structure were carried out using NIH3T3 cells in accordance with the procedure described below, and the cell numbers after 7 days of cell culture were evaluated by WST-1 measurement. First, a fiber sheet structure was punched out with a diameter of 14 mm punch, wrapped in an aluminum pack, and sterilized with electron beams. The sterilized fiber sheet structure was placed on an MPC-coated 24-well plate with the cell-mounting front surface facing up, and 1 mL of phosphate-buffered saline was added and left to stand for 10 minutes to allow the fiber sheet structure to swell. After swelling, the phosphate buffered saline was removed using a 1000 mL pipette, and 1 mL of a cell suspension at $1.5 \times 10^4$ cells/mL was seeded. The cell suspension was prepared in accordance with the following procedure. The medium was removed from a T225 flask in which cells had been previously cultured in a $CO_2$ incubator (37°C, $CO_2$ concentration 5%), the flask was washed twice with 22.5 mL of phosphate-buffered saline, then 22.5 mL of 0.25% trypsin was added to the flask, and the flask was left to stand in the $CO_2$ incubator for 5 minutes. The detachment of the cells was confirmed with a microscope, and then collected in a centrifuge tube. After prewashing was performed with 22.5 mL of medium, centrifugal processing (220 g for 3 minutes) was performed and the supernatant was removed. The obtained cell pellet was loosened with a medium, the cell number was measured using a Muse cell analyzer (0500-3115), and the cell suspension was adjusted to be a cell concentration of $1.5 \times 10^4$ cells/mL. The medium was prepared by adding 53 mL of FBS, 1/1000 volume of kanamycin, and 1/100 volume of a penicillin-streptomycin -amphotericin B suspension to 1000 mL of DMEM medium. After seeding, the cells were placed in a $CO_2$ incubator (MCO-170AICUVH-PJ) (37°C, $CO_2$ concentration 5%) to initiate culture. After 3 and 6 days, the medium was replaced by removing it with a 1000 mL pipette and adding 1 mL of medium again. On the 7th day, 100 μL of WST-1 was added and the mixture was placed in a $CO_2$ incubator (37°C, $CO_2$ concentration 5%) for 3 hours, then 300 μL of the mixture was transferred to a 96-well plate, and the cell number was evaluated by measuring the absorbance (450 nm, reference wavelength 630 nm) using a plate reader (VERSA Max). Note that, a calibration curve for evaluating cell number was created by adding 100 μL of WST-1 to suspensions adjusted to $0.5 \times 10^4$, $1.0 \times 10^4$, $2.0 \times 10^4$, $3.0 \times 10^4$, $4.0 \times 10^4$, $6.0 \times 10^4$, and $8.0 \times 10^4$ cells/ml using the same method for preparing the cell suspension described above, and the suspensions were left in a $CO_2$ incubator (37°C, $CO_2$ concentration 5%) for 3 hours, then 300 μL of the suspensions was transferred to a 96-well plate, and absorbance (450 nm, reference wavelength 630 nm) was measured using a plate reader.

[Cell adhesiveness of fiber sheet structure]

The cell adhesiveness was evaluated by statically culturing NIHT3T3 cells on the structure for 7 days and observing the fluorescently labeled cells under a fluorescence microscope to determine the degree of adhesiveness of the cells.

Excellent: Two-dimensional cell growth adhering to the structure is observed, and the cells are immobilized on the surface of the structure.
Good: Observed are mostly two-dimensional cell growth adhering to the structure, and partly cell aggregations grown three-dimensionally and loosely adhering to the surface of the structure.
Average: Observed are mostly cell aggregations grown three-dimensionally and loosely adhering to the surface of the structure.
Poor: No adhesion is observed between the structure and the cells.

[Cell adhesiveness and cell cultivatability of higher order structure and laminated higher order structure]

The cell adhesiveness and cell cultivatability of the higher order structure and laminated structure were evaluated by statically culturing HeLa cells on the structure for three days and observing the fluorescently labeled cells under a fluorescence microscope. The higher order structure and the laminated structure were placed on an MPC-coated 12-well plate, and medium was added to swell the structures. The medium used was D-MEM/10% FBS and penicillin-streptomycin. After swelling, excess medium was discarded, and $2 \times 10^6$ /ml HeLa cell suspension was seeded at 40 $\mu$l/well. To the structure, 1 ml/well of the medium was added, and the structure was incubated at 37°C in a 5% $CO_2$ environment for 3 days. After incubation, the supernatant was discarded, and the cells were washed with 1 ml/well of phosphate buffered saline. After washing, OPTI-MEM medium containing 2 $\mu$M Calcein AM, a fluorescent reagent, was added at 1 ml/well. The cells were cultured at 37°C in a 5% $CO_2$ environment for 2 hours, and cell adhesion and cell cultivatability were evaluated by qualitative observation using a fluorescent microscope.
The cell adhesiveness was evaluated based on the degree of adhesion of the cells adhered to the higher order structure and the laminated higher order structure.

Excellent: Two-dimensional cell growth adhering to the structure is observed, and the cells are immobilized on the surface of the structure.
Good: Observed are mostly two-dimensional cell growth adhering to the structure, and partly cell aggregations grown three-dimensionally and loosely adhering to the surface of the structure.
Average: Observed are mostly cell aggregations grown three-dimensionally and loosely adhering to the surface of the structure.
Poor: No adhesion is observed between the structure and the cells.
Cell cultivatability was evaluated by qualitative observation of luminescence from cells stained with a fluorescent reagent under a fluorescent microscope.
Excellent: Strong fluorescence is observed over the entire surface of the structure.
Good: Strong fluorescence is observed from the part other than the gaps in the structure (the mesh of the knitted fabric).
Average: Weak fluorescence is observed from the part other than the gaps in the structure (the mesh of the knitted fabric).
Poor: No fluorescence was observed.

[Production Example 1]

To 50 wt% of gelatin (pigskin gelatin type A, manufactured by Nitta Gelatin Inc.) having a jelly strength of 293 g, was added 50 wt% of water and left to stand for 30 minutes, and then the mixture was kneaded and degassed under vacuum type deaeration mixer (ARV-310P, manufactured by Thinky Corporation) to prepare a gelatin dope for spinning. The sol-gel transition temperature of the gelatin dope was 37°C.
The prepared gelatin dope was heated to 50°C and extruded fibrously at a temperature of 50°C from a spinneret having 6 holes with a hole diameter of 150 $\mu$m (perfect circle), the extruded gelatin fiber was conveyed at 30 m/min along the peripheral surface of a conveying roller installed 1.8 m from the extrusion port, and wound up around a bobbin by a winding roller to obtain a gelatin fiber bundle precursor made of 6 gelatin fibers. The extruded gelatin fibers were air-dried during conveyance, and oily agent (a mixed solution of 10% ethylene glycol and 90% ethanol) was applied thereto, and then the fibers were wound up around a bobbin. The moisture content of the gelatin fibers on arriving at the conveying roller was 15 wt%. The moisture content of the gelatin fiber bundle precursor was 13 wt%, no welding between the fiber bundles was observed, and the unwindability from the bobbin was also good. In addition, the fineness of the wound gelatin fiber bundle precursor was 48 dtex, and the tensile strength and tensile elongation of the gelatin fibers taken out from the gelatin fiber bundle precursor were 1.1 cN/dtex and 74%, respectively.

The gelatin fiber bundle precursor wound around the bobbin was heat-treated in air at 140°C for 24 hours to obtain a water-insolubilized gelatin fiber bundle. The fineness of the gelatin fiber bundle was 48 dtex, the tensile strength and the tensile elongation were 1.2 cN/dtex and 84%, respectively, and the fineness of a gelatin fiber was 8 dtex. The gelatin fiber bundle reached the upper yield point (upper yield stress: 0.85 cN/dtex) at an elongation of 5% and the lower yield point (lower yield stress: 0.80 cN/dtex) at an elongation of 15%, and then at an elongation of 84%, broke (1.2 cN/dtex). Furthermore, the weight loss rate of the gelatin fiber bundle when immersed in phosphate buffered saline at 50°C for 4 hours was 18%, and the swelling ratio was 240%.

Next, a higher order structure was obtained using the gelatin fiber bundle with a circular knitting machine (model number: MR-1, manufactured by Maruzen Sangyo Co., Ltd.). The weight loss rate of the higher order structure when immersed in phosphate buffered saline at 50°C for 4 hours was 18%, and 1.2% of the 18% weight loss rate was due to the oily agent. In addition, the thickness of the higher order structure was 83 $\mu$m, and the porosity was 91%.

[Production Example 2]

In the same manner as in Production Example 1, a gelatin fiber bundle precursor (fineness 48 dtex) made of six gelatin fibers before heat treatment was prepared. This gelatin fiber bundle precursor was used to produce a knitted fabric with a circular knitting machine. Next, the gelatin fiber bundles were insolubilized by heat treatment in air at 140°C for 24 hours to obtain a higher order structure made of gelatin fiber bundles. The tensile strength and tensile elongation of the gelatin fiber bundle obtained by spreading this higher order structure were 1.2 cN/dtex and 84%, respectively, and the fineness of a gelatin fiber was 8 dtex. The gelatin fiber bundle reached the upper yield point (upper yield stress: 0.85 cN/dtex) at an elongation of 5% and the lower yield point (lower yield stress: 0.80 cN/dtex) at an elongation of 15%, and then at an elongation of 84%, broke (1.2 cN/dtex).

Furthermore, the weight loss rate of the gelatin fiber bundle when immersed in phosphate buffered saline at 50°C for 4 hours was 18%, and the swelling ratio was 240%. The weight loss rate of the higher order structure when immersed in phosphate buffered saline at 50°C for 4 hours was 18%. In addition, the thickness of the higher order structure was 83 $\mu$m, and the porosity was 91%.

[Production Example 3]

In the same manner as in Production Example 1, a gelatin fiber bundle precursor made of six gelatin fibers before heat treatment was prepared. This gelatin fiber bundle precursor was used to produce a knitted fabric with a circular knitting machine. Next, the knitted fabric was immersed in a 12.5% aqueous glutaraldehyde solution for 5 minutes, washed with water, dried, and water-insolubilized to obtain a higher order structure made of gelatin fiber bundles. The tensile strength and tensile elongation of the gelatin fiber obtained by spreading this higher order structure were 0.8 cN/dtex and 65%, respectively, and the fineness of a gelatin fiber was 8 dtex. The gelatin fiber bundle reached the upper yield point (upper yield stress: 0.8 cN/dtex) at an elongation of 4% and the lower yield point (lower yield stress: 0.70 cN/dtex) at an elongation of 15%, and then at an elongation of 65%, broke (0.78 cN/dtex).

Furthermore, the weight loss rate of the gelatin fiber bundle when immersed in phosphate buffered saline at 50°C for 4 hours was 9%, and the swelling ratio was 138%. The weight loss rate of the higher order structure when immersed in phosphate buffered saline at 50°C for 4 hours was 9%. In addition, the thickness of the higher order structure was 83 $\mu$m, and the porosity was 91%.

[Production Example 4]

A gelatin fiber bundle precursor made of 24 gelatin fibers before heat treatment was produced in the same manner as in Example 1, except that the yarn extruded from a spinneret having 24 holes with a hole diameter of 150 $\mu$m (perfect circular) was conveyed at 90 m/min. The moisture content of the gelatin fibers on arriving at the conveying rollers was 13 wt%. The moisture content of the gelatin fiber bundle precursor was 10 wt%, no welding between the fiber bundles was observed, and the unwindability from the bobbin was also good. In addition, the fineness of the wound gelatin fiber bundle precursor was 133.6 dtex, and the tensile strength and tensile elongation of the gelatin fibers were 1.6 cN/dtex and 50%, respectively. This gelatin fiber bundle precursor was used to produce a knitted fabric with a circular knitting machine. Next, the gelatin fiber bundles were insolubilized by heat treatment in air at 140°C for 24 hours to obtain a higher order structure made of gelatin fiber bundles. The tensile strength and tensile elongation of the gelatin fiber bundle obtained by spreading this higher order structure were 1.6 cN/dtex and 50%, respectively, and the fineness of a gelatin fiber was 5.6 dtex. The gelatin fiber bundle reached the upper yield point (upper yield stress: 1.2 cN/dtex) at an elongation of 5% and the lower yield point (lower yield stress: 1.15 cN/dtex) at an elongation of 15%, and then at an elongation of 50%, broke (1.6 cN/dtex). Furthermore, the weight loss rate of the gelatin fiber bundle when immersed in phosphate buffered saline at 50°C for 4 hours was 17%, and the swelling ratio was 230%.

The weight loss rate of the higher order structure when immersed in phosphate buffered saline at 50°C for 4 hours was 19%. In addition, the thickness of the higher order structure was 132 $\mu$m, and the porosity was 84%.

[Production Example 5]

A higher order structure made of gelatin fiber bundles was produced in the same manner as in Production Example 1, except that 'heat-treated in air at 140°C for 24 hours' was changed to 'heat-treated under vacuum at 180°C for 6 hours'. The fineness of the gelatin fiber bundle was 46 dtex, the tensile strength and the tensile elongation were 1.5 cN/dtex and 62%, respectively, and the fineness of a gelatin fiber was 8 dtex. The gelatin fiber bundle reached the upper yield point (upper yield stress: 1.15 cN/dtex) at an elongation of 5% and the lower yield point (lower yield stress: 1.05 cN/dtex) at an elongation of 15%, and then at an elongation of 62%, broke (1.5 cN/dtex). Furthermore, the weight loss rate of the gelatin fiber bundle when immersed in phosphate buffered saline at 50°C for 4 hours was 9%, and the swelling ratio was 138%. Furthermore, the weight loss rate of the higher order structure when immersed in phosphate buffered saline at 50°C for 4 hours was 9%, and 1.2% of the 9% weight loss rate was due to the oily agent. In addition, the thickness of the higher order structure was 83 $\mu$m, and the porosity was 91%.

[Production Example 6]

A gelatin fiber bundle precursor made of 15 gelatin fibers before heat treatment was produced in the same manner as in Example 1, except that the yarn extruded from a spinneret having 15 holes with a hole diameter of 150 $\mu$m (perfect circular) was conveyed at 60 m/min. The fineness of the wound gelatin fiber bundle precursor was 58.6 dtex, and the tensile strength and tensile elongation of the gelatin fibers were 1.1 cN/dtex and 77%, respectively. A thermoplastic resin fiber bundle made of polyglycolic acid having 15 filaments and a fineness of 35.0 dtex was spirally wound around this gelatin fiber bundle precursor and twisted to produce a doubled yarn bundle precursor having 30 filaments and a fineness of 93.6 dtex. This doubled yarn bundle precursor was used to produce a knitted fabric with a circular knitting machine. Next, the gelatin fiber bundles were insolubilized by heat treatment in air at 140°C for 24 hours to obtain a higher order structure made of doubled yarn bundles containing gelatin fiber bundles and thermoplastic resin fiber bundles. The tensile strength and tensile elongation of the doubled yarn bundle, obtained by spreading this higher order structure, containing gelatin fiber bundles and thermoplastic resin fiber bundles were 7.2 cN/dtex and 35%, respectively. Note that, the gelatin fiber bundle obtained by heat treating the gelatin fiber bundle precursor in air at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex). Furthermore, the weight loss rate of the doubled yarn bundle, of gelatin fiber bundles and thermoplastic resin fiber bundles, immersed in phosphate buffered saline at 50°C for 4 hours was 12%, and the swelling ratio was 145%. The weight loss rate of the higher order structure when immersed in phosphate buffered saline at 50°C for 4 hours was 12%. The thickness of the higher order structure was 125 $\mu$m, and the porosity was 87%.

[Production Example 7]

A higher order structure made of a doubled yarn bundle including a gelatin fiber bundle and a thermoplastic resin fiber bundle was produced in the same manner as in Production Example 6, except that 'a thermoplastic resin fiber bundle made of polyglycolic acid having 15 filaments and a fineness of 35.0 dtex was spirally wound around this gelatin fiber bundle precursor and twisted' was changed to 'this gelatin fiber bundle was spirally wound around a thermoplastic resin fiber bundle made of polyglycolic acid having 15 filaments and a fineness of 35.0 dtex and twisted'. The doubled yarn bundle precursor had 30 filaments, and a fineness of 93.6 dtex. The tensile strength and tensile elongation of the doubled yarn bundle, obtained by spreading this higher order structure, containing gelatin fiber bundles and thermoplastic resin fiber bundles were 7.2 cN/dtex and 35%, respectively. Note that, the gelatin fiber bundle obtained by heat treating the gelatin fiber bundle precursor in air at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex). Furthermore, the weight loss rate of the doubled yarn bundle, of gelatin fiber bundles and thermoplastic resin fiber bundles, immersed in phosphate buffered saline at 50°C for 4 hours was 12%, and the swelling ratio was 145%. The weight loss rate of the higher order structure when immersed in phosphate buffered saline at 50°C for 4 hours was 12%. The thickness of the higher order structure was 125

μm, and the porosity was 87%.

[Production Example 8]

A higher order structure made of doubled yarn bundles containing gelatin fiber bundles and thermoplastic resin fiber bundles was produced in the same manner as in Production Example 7, except that 'heat-treated in air at 140°C for 24 hours' was changed to 'heat-treated under vacuum at 160°C for 12 hours'. The tensile strength and tensile elongation of the doubled yarn bundle, obtained by spreading this higher order structure, containing gelatin fiber bundles and thermoplastic resin fiber bundles were 6.8 cN/dtex and 32%, respectively. Note that, the gelatin fiber bundle obtained by heat treating the gelatin fiber bundle precursor under vacuum at 160°C for 12 hours had a fineness of 58.6 dtex, a tensile strength of 1.3 cN/dtex and a tensile elongation of 65%, and the fineness of a gelatin fiber was 3.9 dtex. The gelatin fiber bundle reached the upper yield point (upper yield stress: 1.0 cN/dtex) at an elongation of 5%, and stretched from the lower yield point (lower yield stress: 0.95 cN/dtex) at an elongation of 15%, and then at an elongation of 65%, broke (1.3 cN/dtex). Furthermore, the weight loss rate of the doubled yarn bundle, of gelatin fiber bundles and thermoplastic resin fiber bundles, immersed in phosphate buffered saline at 50°C for 4 hours was 9%, and the swelling ratio was 135%. The weight loss rate of the higher order structure when immersed in phosphate buffered saline at 50°C for 4 hours was 9%. The thickness of the higher order structure was 125 μm, and the porosity was 87%.

[Production Example 9]

A gelatin fiber bundle precursor made of 15 gelatin fibers before heat treatment was produced in the same manner as in Production Example 1, except that 'a spinneret having 6 holes with a hole diameter of 150 μm (perfect circle)' was changed to 'a spinneret having 15 holes with a hole diameter of 150 μm (perfect circle)', and 'at 30 m/min' was changed to 'at 60 m/min'. The fineness of the wound gelatin fiber bundle precursor was 58.6 dtex, and the tensile strength and tensile elongation of the gelatin fibers taken out from the gelatin fiber bundle precursor were 1.1 cN/dtex and 77%, respectively. Note that, the moisture content of the gelatin fibers on arriving at the conveying rollers was 12 wt%. The moisture content of the gelatin fiber bundle precursor was 10 wt%, no welding between the fiber bundles was observed, and the unwindability from the bobbin was also good.
Next, a thermoplastic resin fiber bundle made of polyglycolic acid having 15 filaments and a fineness of 35.0 dtex was, while being unwound at 30 m/min, washed in an aqueous solution bath containing 40 wt% of ethanol to remove the oily agent, and then dried by contact with a roller heated at 70°C, subsequently, the bundle was immersed in an aqueous collagen solution of Cell Matrix (Type I-C) manufactured by Nitta Gelatin Inc. and dried to obtain a collagen-coated thermoplastic resin fiber bundle. The collagen-coated thermoplastic resin fiber bundle having 15 filaments and a fineness of 35 dtex was spirally wound around this gelatin fiber bundle precursor and twisted to produce a doubled yarn bundle precursor having 30 filaments and a fineness of 93.6 dtex. This doubled yarn bundle precursor was used to produce a knitted fabric with a circular knitting machine. Next, the gelatin fiber bundles were insolubilized by heat treatment in air at 140°C for 24 hours to obtain a higher order structure made of doubled yarn bundles containing gelatin fiber bundles and thermoplastic resin fiber bundles. The tensile strength and tensile elongation of the doubled yarn bundle, obtained by spreading this higher order structure, containing gelatin fiber bundles and thermoplastic resin fiber bundles were 7.2 cN/dtex and 35%, respectively. Note that, the gelatin fiber bundle obtained by heat treating the gelatin fiber bundle precursor in air at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex). Furthermore, the weight loss rate of the doubled yarn bundle, of gelatin fiber bundles and thermoplastic resin fiber bundles, immersed in phosphate buffered saline at 50°C for 4 hours was 12%, and the swelling ratio was 145%. The weight loss rate of the higher order structure when immersed in phosphate buffered saline at 50°C for 4 hours was 12%. The thickness of the higher order structure was 125 μm, and the porosity was 87%.

[Production Example 10]

A higher order structure made of doubled yarn bundles containing gelatin fiber bundles and thermoplastic resin fiber bundles was obtained in the same manner as in Production Example 9, except that 'made of polyglycolic acid having 15 filaments and a fineness of 35.0 dtex' was changed to 'made of polylactic acid having 15 filaments and a fineness of 33.0 dtex'. The doubled yarn bundle precursor had 30 filaments, and a fineness of 91.6 dtex. The tensile strength and tensile elongation of the doubled yarn bundle, obtained by spreading this higher order structure, containing gelatin fiber bundles and thermoplastic resin fiber bundles were 5.5 cN/dtex and 36%, respectively. Note that, the gelatin fiber bundle obtained by heat treating the gelatin fiber bundle precursor in air at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex). Furthermore, the weight loss rate of the doubled yarn bundle, of gelatin fiber bundles and thermoplastic resin fiber bundles, immersed in phosphate buffered saline at 50°C for 4 hours was 12%, and the swelling ratio was 145%. The weight loss rate of the higher order structure when immersed in phosphate buffered saline at 50°C for 4 hours was 12%. The thickness of the higher order structure was 125 μm, and the porosity was 89%.

[Production Example 11]

The higher order structure obtained in Production Example 6 was attached to the base of an electrospinning apparatus (NANON-04, manufactured by MECC CO., LTD.), and a gelatin dope prepared by dissolving 20 wt% (of total weight of the dope) of gelatin in a 30 wt% acetic acid aqueous solution was electrospun for 1 minute under conditions of room temperature, voltage of 20 kV, discharge rate of 0.3 cc/min, and distance from the nozzle tip to the higher order structure of 10 cm, to produce a laminated higher order structure precursor made of a gelatin nonwoven fabric and the higher order structure. This laminated higher order structure precursor was dried under vacuum at room temperature for 24 hours, then pressed at 800 kPa for 60 seconds while applying a temperature of 70°C on the surface of the gelatin nonwoven fabric side, and then heat-treated under vacuum at 160°C for 12 hours to obtain a laminated higher order structure in which the gelatin nonwoven fabric was laminated on the higher order structure. The tensile strength and tensile elongation of the doubled yarn bundle, obtained by spreading this laminated higher order structure, containing gelatin fiber bundles and thermoplastic resin fiber bundles were 6.8 cN/dtex and 32%, respectively. Furthermore, the weight loss rate of the doubled yarn bundle, of gelatin fiber bundles and thermoplastic resin fiber bundles, immersed in phosphate buffered saline at 50°C for 4 hours was 9%, and the swelling ratio was 135%. The weight loss rate of the laminated higher order structure when immersed in phosphate buffered saline at 50°C for 4 hours was 9%, and 1.0% of the 9% weight loss rate was due to the oily agent. The thickness of the laminated higher order structure was 125 μm, and the porosity was 87%.

[Production Example 12]

A laminated higher order structure in which a gelatin nonwoven fabric was laminated with a higher order structure was obtained in the same manner as in Production Example 11, except that 'heat-treated under vacuum at 160°C for 12 hours' was changed to 'heat-treated in air at 140°C for 24 hours'. The tensile strength and tensile elongation of the doubled yarn bundle, obtained by spreading this laminated higher order structure, containing gelatin fiber bundles and thermoplastic resin fiber bundles were 7.2 cN/dtex and 35%, respectively. Furthermore, the weight loss rate of the doubled yarn bundle, of gelatin fiber bundles and thermoplastic resin fiber bundles, immersed in phosphate buffered saline at 50°C for 4 hours was 12%, and the swelling ratio was 145%.
The weight loss rate of the laminated higher order structure when immersed in phosphate buffered saline at 50°C for 4 hours was 12%, and 1.0% of the 12% weight loss rate was due to the oily agent. The thickness of the laminated higher order structure was 125 μm, and the porosity was 87%.

[Production Example 13]

The higher order structure obtained in Production Example 6 was attached to the base of an electrospinning apparatus (NANON-04, manufactured by MECC CO., LTD.), and a collagen dope prepared by dissolving 10 wt% (of total weight of the dope) of collagen in a 84 wt% acetic acid aqueous solution was electrospun for 1 minute under conditions of room temperature, voltage of 20 kV, discharge rate of 0.3 cc/min, and distance from the nozzle tip to the higher order structure of 10 cm, to produce a laminated higher order structure precursor made of a collagen nonwoven fabric and the higher order structure. This laminated higher order structure precursor was dried under vacuum at room temperature for 24 hours, then pressed at 80°C and 800 kPa for 60 seconds, and then heat-treated under vacuum at 140°C for 24 hours to obtain a laminated higher order structure in which the collagen nonwoven fabric was laminated on the higher order structure. The tensile strength and tensile elongation of the doubled yarn bundle, obtained by spreading this laminated higher order structure, containing gelatin fiber bundles and thermoplastic resin fiber bundles were 7.2 cN/dtex and 35%, respectively. Furthermore, the weight loss rate of the doubled yarn bundle, of gelatin fiber bundles and thermoplastic resin fiber bundles, immersed in phosphate buffered saline at 50°C for 4 hours was 12%, and the swelling ratio was 145%.
The weight loss rate of the laminated higher order structure when immersed in phosphate buffered saline at 50°C for 4 hours was 13%, and 1.0% of the 13% weight loss rate was due to the oily agent. The thickness of the laminated higher order structure was 125 μm, and the porosity was 87%.

[Production Example 14]

A gelatin monohole yarn precursor before heat treatment was produced in the same manner as in Example 1, except that the yarn extruded from a monohole spinneret with a hole diameter of 150 $\mu$m (perfect circular) was conveyed at 10 m/min. The moisture content of the wound gelatin monohole yarn precursor was 17 wt%, no welding between the fibers was observed, and the unwindability from the bobbin was also good. In addition, the fineness of the wound gelatin monohole yarn precursor was 128 dtex, and the tensile strength and tensile elongation of the gelatin fiber (gelatin monohole yarn precursor) were 0.6 cN/dtex and 63%, respectively.

The gelatin monohole yarn precursor wound around the bobbin was heat-treated in air at 140°C for 24 hours to obtain a water-insolubilized gelatin monohole yarn. The insolubilized gelatin monohole yarn had a fineness of 128 dtex, a tensile strength of 0.7 cN/dtex, and a tensile elongation of 122%. Furthermore, the weight loss rate of the gelatin monohole yarn when immersed in phosphate buffered saline at 50°C for 4 hours was 19%, and the swelling ratio was 280%.

Next, production of a knitted fabric was attempted using the gelatin monohole yarn with a circular knitting machine, resulting in no knitted fabric obtained due to many missing stitches.

[Production Example 15]

Production of a knitted fabric was attempted using a commercially available PET monohole yarn having a fineness of 110 dtex with a circular knitting machine, resulting in no knitted fabric obtained due to many missing stitches.

[Production Example 16]

A gelatin monohole yarn precursor before heat treatment was produced in the same manner as in Example 1, except that the yarn extruded from a monohole spinneret with a hole diameter of 150 $\mu$m (perfect circular) was conveyed at 30 m/min. The moisture content of the gelatin monohole yarn precursor was 15 wt%, no welding between the fibers was observed, and the unwindability from the bobbin was also good. In addition, the fineness of the wound gelatin monohole yarn precursor was 8 dtex, and the tensile strength and tensile elongation of the gelatin fiber (gelatin monohole yarn precursor) were 1.0 N/cm and 72%, respectively.

The gelatin monohole yarn precursor wound around the bobbin was heat-treated in air at 140°C for 24 hours to obtain a water-insolubilized gelatin monohole yarn. The gelatin monohole yarn had a fineness of 8 dtex, a tensile strength of 1.2 cN/dtex, and a tensile elongation of 84%. Furthermore, the weight loss rate of the gelatin monohole yarn when immersed in phosphate buffered saline at 50°C for 4 hours was 18%. Next, production of a knitted fabric was attempted using the gelatin monohole yarn with a circular knitting machine, resulting in no knitted fabric obtained due to many yarn breakages caused by poor yarn strength.

[Production Example 17]

A higher order structure was produced with a circular knitting machine using a thermoplastic resin fiber bundle made of polylactic acid having 15 filaments and a fineness of 33.0 dtex. The tensile strength and tensile elongation of the thermoplastic resin fiber bundle obtained by spreading this higher order structure were 4.5 cN/dtex and 33%, respectively. Furthermore, the weight loss rate of the thermoplastic resin fiber bundle when immersed in phosphate buffered saline at 50°C for 4 hours was 0%, and the swelling ratio was 100%. The weight loss rate of the higher order structure when immersed in phosphate buffered saline at 50°C for 4 hours was 0%. The thickness of the higher order structure was 78 $\mu$m, and the porosity was 85%.

[Production Example 18]

Using the doubled yarn bundle precursor of Production Example 6, a double knit (second higher order structure), having a thickness of 2040 $\mu$m, an average pore size of 95 $\mu$m, and a porosity of 94%, was produced with WHOLEGARMENT (registered trademark) (SWG (registered trademark) 041N2) by laminating two layers of plain stitch. This double knit was heat treated under vacuum at 140°C for 24 hours for insolubilization, then heated at 70°C on one side of the double knit and pressed at 800 kPa for 60 seconds to obtain a fiber sheet structure. The tensile strength and tensile elongation of the doubled yarn bundle, obtained by spreading this fiber sheet structure, containing gelatin fiber bundles and thermoplastic resin fiber bundles were 7.2 cN/dtex and 35%, respectively. Furthermore, the weight loss rate of the doubled yarn bundle when immersed in phosphate buffered saline at 50°C for 4 hours was 12%, and the swelling ratio was 145%. Note that, the gelatin fiber bundle obtained by heat treating the gelatin fiber bundle precursor under vacuum at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex).

[Production Example 19]

Using the doubled yarn bundle precursor of Production Example 6, a double knit (second higher order structure), having a thickness of 1670 μm, an average pore size of 127 μm, and a porosity of 93%, was produced with WHOLEGARMENT (registered trademark) (SWG (registered trademark) 041N2) by laminating two layers of plain stitch. This double knit was heat treated under vacuum at 140°C for 24 hours for insolubilization, then heated at 60°C on one side and pressed at 700 kPa for 60 seconds to obtain a fiber sheet structure. The tensile strength and tensile elongation of the doubled yarn bundle, obtained by spreading this fiber sheet structure, containing gelatin fiber bundles and thermoplastic resin fiber bundles were 7.2 cN/dtex and 35%, respectively. Furthermore, the weight loss rate of the doubled yarn bundle when immersed in phosphate buffered saline at 50°C for 4 hours was 12%, and the swelling ratio was 145%. Note that, the gelatin fiber bundle obtained by heat treating the gelatin fiber bundle precursor under vacuum at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex).

[Production Example 20]

Using the doubled yarn bundle precursor of Production Example 6, a double knit (second higher order structure), having a thickness of 1940 μm, an average pore size of 150 μm, and a porosity of 95%, was produced with WHOLEGARMENT (registered trademark) (SWG (registered trademark) 041N2) by laminating two layers of plain stitch. This double knit was heat treated under vacuum at 140°C for 24 hours for insolubilization, then heated at 70°C on one side and pressed at 800 kPa for 60 seconds to obtain a fiber sheet structure. The tensile strength and tensile elongation of the doubled yarn bundle, obtained by spreading this second higher order structure, containing gelatin fiber bundles and thermoplastic resin fiber bundles were 7.2 cN/dtex and 35%, respectively. Furthermore, the weight loss rate of the doubled yarn bundle when immersed in phosphate buffered saline at 50°C for 4 hours was 12%, and the swelling ratio was 145%. Note that, the gelatin fiber bundle obtained by heat treating the gelatin fiber bundle precursor under vacuum for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex).

[Production Example 21]

Using the doubled yarn bundle precursor of Production Example 7, a double knit (second higher order structure), having a thickness of 2040 μm, an average pore size of 95 μm, and a porosity of 94%, was produced with WHOLEGARMENT (registered trademark) (SWG (registered trademark) 041N2) by laminating two layers of plain stitch. This double knit was heat treated under vacuum at 140°C for 24 hours for insolubilization, then heated at 70°C on one side and pressed at 800 kPa for 60 seconds to obtain a fiber sheet structure. The tensile strength and tensile elongation of the doubled yarn bundle, obtained by spreading this fiber sheet structure, containing gelatin fiber bundles and thermoplastic resin fiber bundles were 7.2 cN/dtex and 35%, respectively. Furthermore, the weight loss rate of the doubled yarn bundle when immersed in phosphate buffered saline at 50°C for 4 hours was 12%, and the swelling ratio was 145%. Note that, the gelatin fiber bundle obtained by heat treating the gelatin fiber bundle precursor under vacuum at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex).

[Production Example 22]

A doubled yarn bundle precursor containing four gelatin fiber bundle precursors and four thermoplastic resin fiber bundles was produced using four doubled yarn bundle precursors and four thermoplastic resin fiber bundles of Production Example 6, and using the doubled yarn bundle precursor, a single knit (second higher order structure) having a thickness of 940 μm, an average pore size of 125 μm, and a porosity of 94% was produced by rib stitch with a WHOLEGARMENT (registered trademark) (SWG (registered trademark) 041N2). This single knit was insolubilized under vacuum at 140°C for 24 hours, and then heated at 70°C on one side and pressed at 800 kPa for 60 seconds to obtain a fiber sheet structure. The tensile strength and tensile elongation of the doubled yarn bundle, obtained by spreading this fiber sheet structure, containing gelatin fiber bundles and thermoplastic resin fiber bundles were 7.2 cN/dtex and 35%, respectively. Furthermore, the weight loss rate of the doubled yarn bundle when immersed in phosphate buffered saline at 50°C for 4 hours was 12%, and the swelling ratio was 145%. Note that, the gelatin fiber bundle obtained by heat treating the gelatin fiber bundle precursor in air at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex).

[Production Example 23]

The double knit (second higher order structure) in Production Example 19 was attached to the base of an electrospinning apparatus (NANON-04, manufactured by MECC CO., LTD.), and a gelatin dope prepared by dissolving 20 wt% (of total weight of the dope) of gelatin in a 30 wt% acetic acid aqueous solution was electrospun for 1 minute under conditions of room temperature, voltage of 20 kV, discharge rate of 0.3 cc/min, and distance from the nozzle tip to the second higher order structure of 10 cm, to produce a second laminated higher order structure made of a gelatin nonwoven fabric and the second higher order structure. This second laminated higher order structure was heat treated under vacuum at 140°C for 24 hours for insolubilization, and then one side was heated to 60°C and pressed at 700 kPa for 60 seconds to obtain a fiber sheet structure. The tensile strength and tensile elongation of the doubled yarn bundle, obtained by spreading this fiber sheet structure, containing gelatin fiber bundles and thermoplastic resin fiber bundles were 7.2 cN/dtex and 35%, respectively. Furthermore, the weight loss rate of the doubled yarn bundle when immersed in phosphate buffered saline at 50°C for 4 hours was 12%, and the swelling ratio was 145%. Note that, the gelatin fiber bundle obtained by heat treating the gelatin fiber bundle precursor in air at 140°C for 24 hours had a fineness of 58.6 dtex, a tensile strength of 1.2 cN/dtex and a tensile elongation of 79%, and the fineness of a gelatin fiber was 3.9 dtex. The gelatin fiber bundle reached the upper yield point (upper yield stress: 0.85 cN/dtex) at an elongation of 5%, and the lower yield point (lower yield stress: 0.80 cN/dtex) at an elongation of 15%, and then at an elongation of 79%, broke (1.2 cN/dtex).

[Production Example 24]

The single knit (second higher-order structure) in Production Example 22 was attached to the base of an electrospinning apparatus (NANON-04, manufactured by MECC CO., LTD.), and a gelatin dope prepared by dissolving 20 wt% (of total weight of the dope) of gelatin in a 30 wt% acetic acid aqueous solution was electrospun for 1 minute under conditions of room temperature, voltage of 20 kV, discharge rate of 0.3 cc/min, and distance from the nozzle tip to the second higher order structure of 10 cm, to produce a second laminated higher order structure made of a gelatin nonwoven fabric and the second higher order structure. This second laminated higher order structure was heat treated under vacuum at 160°C for 12 hours for insolubilization, and then the surface on the gelatin nonwoven fabric side was heated at 70°C and pressed at 800 kPa for 60 seconds to obtain a fiber sheet structure. The tensile strength and tensile elongation of the doubled yarn bundle, obtained by spreading this fiber sheet structure, containing gelatin fiber bundles and thermoplastic resin fiber bundles were 6.8 cN/dtex and 32%, respectively. Furthermore, the weight loss rate of the doubled yarn bundle when immersed in phosphate buffered saline at 50°C for 4 hours was 9%, and the swelling ratio was 135%. Note that, the gelatin fiber bundle obtained by heat treating the gelatin fiber bundle precursor under vacuum at 160°C for 24 hours had a fineness of 52.5 dtex, a tensile strength of 1.1 cN/dtex and a tensile elongation of 70%, and the fineness of a gelatin fiber was 3.5 dtex. The gelatin fiber bundle reached the upper yield point (upper yield stress: 0.82 cN/dtex) at an elongation of 5% and the lower yield point (lower yield stress: 0.75 cN/dtex) at an elongation of 14%, and then at an elongation of 56%, broke (0.9 cN/dtex).

[Reference Example 1]

The higher order structure obtained in Production Example 1 was evaluated for texture, average friction coefficient, average deviation of friction coefficient, average pore size, proportion of number of pores with a size of 150 μm or more, number of pores, presence or absence of curling, and cell adhesiveness and cell cultivatability using HeLa cells.

[Reference Example 2]

The higher order structure obtained in Production Example 2 was evaluated for texture.

[Reference Example 3]

The higher order structure obtained in Production Example 3 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference Example 4]

The higher order structure obtained in Production Example 4 was evaluated for texture.

[Reference example 5]

The higher order structure obtained in Production Example 5 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 6]

The higher order structure obtained in Production Example 6 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 7]

The higher order structure obtained in Production Example 7 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 8]

The higher order structure obtained in Production Example 8 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 9]

The higher order structure obtained in Production Example 9 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 10]

The higher order structure obtained in Production Example 10 was evaluated for texture, average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 11]

The laminated higher order structure obtained in Production Example 11 was evaluated for average friction coefficient, average deviation of friction coefficient, average pore size, proportion of number of pores with a size of 150 $\mu$m or more, number of pores, presence or absence of curling, and cell adhesiveness and cell cultivatability using HeLa cells.

[Reference example 12]

The laminated higher order structure obtained in Production Example 12 was evaluated for texture, average friction coefficient, average deviation of friction coefficient, average pore size, proportion of number of pores with a size of 150 μm or more, number of pores, presence or absence of curling, and cell adhesiveness and cell cultivatability using HeLa cells.

[Reference example 13]

The laminated higher order structure obtained in Production Example 13 was evaluated for texture, average friction coefficient, average deviation of friction coefficient, average pore size, proportion of number of pores with a size of 150 μm or more, number of pores, presence or absence of curling, and cell adhesiveness and cell cultivatability using HeLa cells.

The evaluation results of Reference Examples 1 to 13 are shown in Tables 1 and 2.

[Example 1]

The fiber sheet structure obtained in Production Example 18 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Example 2]

The fiber sheet structure obtained in Production Example 19 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Example 3]

The fiber sheet structure obtained in Production Example 20 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Example 4]

The fiber sheet structure obtained in Production Example 21 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Example 5]

The fiber sheet structure obtained in Production Example 22 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Example 6]

The fiber sheet structure obtained in Production Example 23 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Example 7]

The fiber sheet structure obtained in Production Example 24 was immersed in phosphate buffered saline at 50°C for 4 hours, and evaluated for the weight loss rate, thickness, porosity, average pore size, proportion of number of pores with a size of 150 μm or more, number of pores, texture, average friction coefficient, average deviation of friction coefficient, presence or absence of curling, and cell adhesiveness and number of cultured cells using NIH3T3 cells.

[Comparative Example 1]

The texture of the fiber in Production Example 14 was evaluated.

[Comparative Example 2]

The texture of the fiber in Production Example 15 was evaluated.

[Comparative Example 3]

The texture of the fiber in Production Example 16 was evaluated.

[Comparative Example 4]

The higher order structure obtained in Production Example 17 was evaluated for texture, average friction coefficient, average deviation of friction coefficient, average pore size, proportion of number of pores with a size of 150 μm or more, number of pores, presence or absence of curling, and cell adhesiveness and cell cultivatability using NIH3T3 cells.

[Comparative Example 5]

A collagen sheet surface of Geistlich Bio-Gide (a two-layer structure of collagen sheet and collagen nonwoven fabric) was evaluated for the average friction coefficient, average deviation of the friction coefficient, presence or absence of curling, and cell adhesiveness and number of cultured cells using NIH3T3 cells. The evaluation results of the examples and comparative examples are shown in Tables 3 and 4.

[Table 1]

| | Production Example | Configuration of higher order structure | | | | | | | | | | | | | | | | | | Configuration of laminated higher order structure | | | | | | |
| | | Configuration of gelatin fiber bundle | | | | | Configuration of thermoplastic resin fiber bundle | | Configuration of doubled yarn bundle | | | | | Weight loss rate (%) | Thickness (μm) | Porosity (%) | Average pore size (μm) | Proportion of number of pores with a size of 150 μm or more (%) | Number of pores (pores/mm³) | Laminated component | Weight loss rate (%) | Thickness (μm) | Porosity (%) | Average pore size (μm) | Proportion of number of pores with a size of 150 μm or more (%) | Number of pores (pores/mm³) |
| | | Number of filaments | Tensile strength (cN/dtex) | Breaking elongation (%) | Weight loss rate (%) | Swelling ratio (%) | Number of filaments | Coating | Number of filaments | Tensile strength (cN/dtex) | Breaking elongation (%) | Weight loss rate (%) | Swelling ratio (%) | | | | | | | | | | | | | |
| Ref Ex 1 | 1 | 6 | 1.2 | 84 | 18 | 240 | - | - | - | - | - | - | - | 18 | 83 | 91 | 185 | 55 | 232 | - | - | - | - | - | - | - |
| Ref Ex 2 | 2 | 6 | 1.2 | 84 | 18 | 240 | - | - | - | - | - | - | - | 18 | 83 | 91 | - | - | - | - | - | - | - | - | - | - |
| Ref Ex 3 | 3 | 6 | 0.8 | 65 | 9 | 138 | - | - | - | - | - | - | - | 9 | 83 | 91 | 180 | 55 | 232 | - | - | - | - | - | - | - |
| Ref Ex 4 | 4 | 24 | 1.6 | 50 | 17 | 230 | - | - | - | - | - | - | - | 19 | 132 | 84 | - | - | - | - | - | - | - | - | - | - |
| Ref Ex 5 | 5 | 6 | 1.5 | 62 | 9 | 138 | - | - | - | - | - | - | - | 9 | 83 | 91 | 185 | 55 | 232 | - | - | - | - | - | - | - |
| Ref Ex 6 | 6 | 15 | 1.2 | 79 | - | - | 15 | - | 30 | 7.2 | 35 | 12 | 145 | 12 | 125 | 87 | 163 | 46 | 257 | - | - | - | - | - | - | - |
| Ref Ex 7 | 7 | 15 | 1.2 | 79 | - | - | 15 | - | 30 | 7.2 | 35 | 12 | 145 | 12 | 125 | 87 | 163 | 46 | 257 | - | - | - | - | - | - | - |
| Ref Ex 8 | 8 | 15 | 1.3 | 65 | - | - | 15 | - | 30 | 6.8 | 32 | 9 | 135 | 9 | 125 | 87 | 163 | 46 | 257 | - | - | - | - | - | - | - |
| Ref Ex 9 | 9 | 15 | 1.2 | 79 | - | - | 15 | collagen | 30 | 7.2 | 35 | 12 | 145 | 12 | 125 | 87 | 163 | 46 | 257 | - | - | - | - | - | - | - |
| Ref Ex 10 | 10 | 15 | 1.2 | 79 | - | - | 15 | collagen | 30 | 5.5 | 36 | 12 | 145 | 12 | 125 | 89 | 163 | 46 | 257 | - | - | - | - | - | - | - |
| Ref Ex 11 | 11 | 15 | 1.2 | 79 | - | - | 15 | - | 30 | 6.8 | 32 | 9 | 135 | 9 | - | - | - | - | - | GNF | 9 | 125 | 87 | 99 | 7 | 720 |
| Ref Ex 12 | 12 | 15 | 1.2 | 79 | - | - | 15 | - | 30 | 7.2 | 35 | 12 | 145 | 12 | - | - | - | - | - | GNF | 12 | 125 | 87 | 99 | 7 | 720 |
| Ref Ex 13 | 13 | 15 | 1.2 | 79 | - | - | 15 | - | 30 | 7.2 | 35 | 12 | 145 | 12 | - | - | - | - | - | CNF | 13 | 125 | 87 | 99 | 7 | 720 |

Reference Example: Ref Ex, GNF: Gelatin nonwoven fabric, CNF: Collagen nonwoven fabric

[Table 2]

| | | Characteristics of higher order structure or laminated higher order structure | | | | | |
|---|---|---|---|---|---|---|---|
| | Texture | Average friction coefficient | Average deviation of surface friction coefficient | Cell adhesiveness | Cell cultivatability | Curl or not |
| Ref Ex 1 | Excellent | 0.27-0.39 | 0.64-1.22 | Average | Average | Yes |
| Ref Ex 2 | Excellent | - | - | - | - | - |
| Ref Ex 3 | Good | 0.22-0.40 | 0.61-1.10 | Good | Good | Yes |
| Ref Ex 4 | Excellent | - | - | - | - | - |
| Ref Ex 5 | Excellent | 0.29-0.42 | 0.66-1.26 | Good | Good | Yes |
| Ref Ex 6 | Excellent | 0.29-0.35 | 1.08-1.27 | Good | Good | Yes |
| Ref Ex 7 | Excellent | 0.23-0.42 | 0.58-1.18 | Average | Average | Yes |
| Ref Ex 8 | Excellent | 0.28-0.38 | 1.02-1.23 | Good | Good | Yes |
| Ref Ex 9 | Excellent | 0.23-0.35 | 0.85-1.27 | Excellent | Good | Yes |
| Ref Ex 10 | Excellent | 0.27-0.38 | 0.96-1.25 | Excellent | Good | Yes |
| Ref Ex 11 | Excellent | 0.25-0.39 | 0.62-1.21 | Excellent | Excellent | Yes |
| Ref Ex 12 | Excellent | 0.26-0.38 | 0.62-1.21 | Good | Excellent | Yes |
| Ref Ex 13 | Excellent | 0.25-0.37 | 0.60-1.16 | Excellent | Excellent | Yes |
| Reference Example: Ref Ex | | | | | | |

[Table 3]

| | Production Example | Configuration of fiber sheet structure | | | | | | | | | | | | | | | | | |
|---|---|---|---|---|---|---|---|---|---|---|---|---|---|---|---|---|---|---|
| | | Configuration of gelatin fiber bundle | | | | Configuration of thermoplastic resin fiber bundle | Configuration of doubled yarn bundle | | | | | | | | | | | |
| | | Number of filaments | Fineness of a gelatin fiber (dtex) | Tensile strength (cN/dtex) | Breaking elongation (%) | Number of filaments | Number of filaments | Number of gelatin fiber bundles | Number of thermoplastic resin fiber bundles | Tensile strength (cN/dtex) | Breaking elongation (%) | Weight loss rate (%) | Swelling ratio (%) | Weight loss rate (%) | Thickness (μm) | Porosity (%) | Average pore size (μm) | Proportion of number of pores with a size of 150 μm or more (%) | Number of pores (pores/mm³) |
| Ex 1 | 18 | 15 | 3.9 | 1.2 | 79 | 15 | 30 | 1 | 1 | 7.2 | 35 | 12 | 145 | 12 | 830 | 83 | 86 | 8 | $2.5 \times 10^3$ |
| Ex 2 | 19 | 15 | 39 | 1.2 | 79 | 15 | 30 | 1 | 1 | 72 | 35 | 12 | 145 | 12 | 830 | 86 | 118 | 18 | $1.7 \times 10^3$ |
| Ex 3 | 20 | 15 | 3.9 | 1.2 | 79 | 15 | 30 | 1 | 1 | 7.2 | 35 | 12 | 145 | 12 | 390 | 73 | 128 | 33 | $1.6 \times 10^3$ |
| Ex 4 | 21 | 15 | 3.9 | 1.2 | 79 | 15 | 30 | 1 | 1 | 7.2 | 35 | 12 | 145 | 12 | 830 | 83 | 86 | 8 | $2.5 \times 10^3$ |
| Ex 5 | 22 | 15 | 3.9 | 1.2 | 79 | 15 | 30 | 4 | 4 | 7.2 | 35 | 12 | 145 | 12 | 520 | 81 | 98 | 22 | $2.1 \times 10^3$ |
| Ex 6 | 23 | 15 | 3.9 | 1.2 | 79 | 15 | 30 | 1 | 1 | 7.2 | 35 | 12 | 145 | 12 | 810 | 86 | 118 | 18 | $1.7 \times 10^3$ |
| Ex 7 | 24 | 15 | 3.9 | 1.2 | 79 | 15 | 30 | 4 | 4 | 6.8 | 32 | 9 | 135 | 9 | 521 | 81 | 98 | 22 | $2.1 \times 10^3$ |
| Comp Ex 1 | 14 | 1 | - | - | - | - | - | - | - | - | - | - | - | - | - | - | - | - | - |
| Comp Ex 2 | 15 | 1 | - | - | - | - | - | - | - | - | - | - | - | - | - | - | - | - | - |
| Comp Ex 3 | 16 | 1 | - | - | - | - | - | - | - | - | - | - | - | - | - | - | - | - | - |
| Comp Ex 4 | 17 | - | - | - | - | 15 | - | - | - | - | - | - | - | 0 | 78 | 85 | 185 | 55 | 232 |
| Comp Ex 5 | - | - | - | - | - | - | - | - | - | - | - | - | - | - | - | - | - | - | - |

Example: Ex, Comparative Example: Comp Ex

[Table 4]

| | | Characteristics of fiber sheet structure | | | | |
|---|---|---|---|---|---|---|
| | Texture | Average friction coefficient | Average deviation of surface friction coefficient | Cell adhesiveness | Number of cultured cells ($\times 10^4$cells/mL) | Curl or not |
| Ex 1 | Excellent | 0.29-0.35 | 0.88-1.12 | Excellent | 33.8 | No |
| Ex 2 | Excellent | 0.29-0.34 | 0.86-1.14 | Excellent | 25.3 | No |
| Ex 3 | Excellent | 0.29-0.35 | 0.88-1.12 | Excellent | 10.2 | No |
| Ex 4 | Excellent | 0.23-0.38 | 0.58-1.08 | Excellent | 33.8 | No |
| Ex 5 | Excellent | 0.29-0.35 | 0.98-1.32 | Excellent | 28.5 | No |
| Ex 6 | Excellent | 0.29-0.34 | 0.88-1.11 | Excellent | 30.6 | No |
| Ex 7 | Excellent | 0.29-0.35 | 0.58-1.08 | Excellent | 33.7 | No |
| Comp Ex 1 | Poor | - | - | - | - | - |
| Comp Ex 2 | Poor | - | - | - | - | - |
| Comp Ex 3 | Poor | - | - | - | - | - |
| Comp Ex 4 | Average | 0.25-0.47 | 0.64-2.43 | Poor | 0.2 | Yes |
| Comp Ex 5 | - | 0.83-1.33 | 1.38-1.78 | Excellent | 33.5 | No |
| Example: Ex, Comparative Example: Comp Ex | | | | | | |

The results shown in Table 4 demonstrate that the fiber sheet structures obtained in the Examples have a good texture, moderate slipperiness, and excellent cell adhesiveness and cultivatability after cell colonization. SEM images confirmed that cells had penetrated the inner layer of the fiber sheet structure and, from the colonized spot, grew as creeping along the fibers and filling in the knots between the fibers.

[Industrial Applicability]

The fiber sheet structure of the present invention is a medical material with excellent mechanical strength and biocompatibility (low antigenicity and high bioabsorbability), and can be suitably used as wound/burn treatment materials and artificial skin matrix materials, etc. It can also be suitably used as a scaffold material for three-dimensional cell culture, which is excellent in promoting cell differentiation, enhancing cell function, and colonizing after transplantation.

**Claims**

1. A fiber sheet structure,

    the fiber sheet structure includes a knit weave and has a cell-mounting front surface and a back surface,
    the average pore size of the back surface is smaller than the average pore size of the cell-mounting front surface,
    the knit weave includes a doubled yarn bundle made of a gelatin fiber bundle and a thermoplastic resin fiber bundle,
    the thickness of the fiber sheet structure is 30 to 5000 $\mu$m,
    the number of gelatin fibers constituting the gelatin fiber bundle is 5 to 60, and the fineness of the gelatin fiber is 0.5 to 10 dtex,
    the doubled yarn bundle has the total number of gelatin fiber bundles and thermoplastic resin fiber bundles of 2 to 20 and the ratio of the number of gelatin fiber bundles to the number of thermoplastic resin fiber bundles of 5:1 to 1:5.

2. The fiber sheet structure according to claim 1, wherein the fiber sheet structure is any one of the following (1) to (4):

    (1) The structure is two-layer laminated, the front surface side layer and the back surface side layer have a knit weave, and the knit weave is a jersey stitch or plain stitch,

(2) The structure is three-layer laminated, the front surface side layer has a knit weave, the back surface side layer is a nonwoven fabric, an intermediate layer between the front surface side layer and the back surface side layer has a knit weave, the knit weave is a jersey stitch or plain stitch, and the nonwoven fabric is a structure containing gelatin fibers,

(3) The structure is two-layer laminated, the front surface side layer has a knit weave, the back surface side layer has a nonwoven fabric, the knit weave is a rib stitch, and the nonwoven fabric is a structure containing gelatin fibers,

(4) A structure with a knit weave consisting of a rib stitch.

3. The fiber sheet structure according to claim 1 or 2, wherein the average pore size of the cell-mounting front surface is 5 to 123 $\mu$m, the number of pores with a size of 150 $\mu$m or more on the cell-mounting front surface is 35% or less, and the number of pores in the knit weave is 600 pores/mm$^3$ or more.

4. The fiber sheet structure according to claim 3, wherein the swelling ratio of the doubled yarn bundle is 110 to 350% when immersed in phosphate buffered saline at 50°C for 4 hours.

5. A fiber sheet structure according to claim 3, wherein the tensile strength of the doubled yarn bundle is 1.0 to 20.0 cN/dtex and the breaking elongation is 20 to 250%.

6. The fiber sheet structure according to claim 3, wherein the weight loss rate of the fiber sheet structure is 3 to 30% when immersed in phosphate buffered saline at 50°C for 4 hours.

## INTERNATIONAL SEARCH REPORT

| International application No. |
|---|
| **PCT/JP2023/017834** |

**A. CLASSIFICATION OF SUBJECT MATTER**

*D01F 4/00*(2006.01)i; *A61L 27/22*(2006.01)i; *A61L 27/60*(2006.01)i; *D02G 3/04*(2006.01)i; *D04B 1/14*(2006.01)i
FI: D01F4/00 A; D04B1/14; D02G3/04; A61L27/60; A61L27/22

According to International Patent Classification (IPC) or to both national classification and IPC

**B. FIELDS SEARCHED**

Minimum documentation searched (classification system followed by classification symbols)

D01F4/00; A61L27/22; A61L2/00-33/18; D02G1/00-3/48; D02J1/00-13/00; D04B1/00-39/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2021/100718 A1 (THE JAPAN WOOL TEXTILE CO., LTD.) 27 May 2021 (2021-05-27) claims | 1-6 |
| A | JP 2010-180482 A (EMPRIE TECHNOLOGY DEVELOPMENT LLC) 19 August 2010 (2010-08-19) claims, paragraph [0009] | 1-6 |
| A | JP 2001-89929 A (KANSAI UNIVERSITY) 03 April 2001 (2001-04-03) claims, paragraphs [0019]-[0022], examples 1-4 | 1-6 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 July 2023** | **25 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/017834**

| Patent document cited in search report | | | Publication date (day/month/year) | Patent family member(s) | | | Publication date (day/month/year) |
|---|---|---|---|---|---|---|---|
| WO | 2021/100718 | A1 | 27 May 2021 | US claims | 2022/0389355 | A1 | |
| | | | | EP | 4063477 | A1 | |
| | | | | CN | 114729308 | A | |
| JP | 2010-180482 | A | 19 August 2010 | US claims, paragraph [0020] | 2010/0190403 | A1 | |
| JP | 2001-89929 | A | 03 April 2001 | (Family: none) | | | |

Form PCT/ISA/210 (patent family annex) (January 2015)

**REFERENCES CITED IN THE DESCRIPTION**

**Patent documents cited in the description**

- JP 2012167397 A
- JP 2005120527 A
- JP 2005163204 A
- JP 2001089929 A